(19) Europäisches Patentamt
European Patent Office
Office européen des brevets

(11) Veröffentlichungsnummer: **0 184 173 B1**

(12) # EUROPÄISCHE PATENTSCHRIFT

(45) Veröffentlichungstag der Patentschrift: **02.02.94**

(51) Int. Cl.⁵: **C07D 493/22**, A01N 43/90, //(C07D493/22,313:00,311:00, 311:00,307:00)

(21) Anmeldenummer: **85115254.6**

(22) Anmeldetag: **02.12.85**

Die Akte enthält technische Angaben, die nach dem Eingang der Anmeldung eingereicht wurden und die nicht in dieser Patentschrift enthalten sind.

(54) **13beta-Milbemycinderivate zur Bekämpfung von Ekto- und Endoparasiten an Pflanzen und Tieren.**

(30) Priorität: **04.12.84 CH 5750/84**

(43) Veröffentlichungstag der Anmeldung:
**11.06.86 Patentblatt 86/24**

(45) Bekanntmachung des Hinweises auf die Patenterteilung:
**02.02.94 Patentblatt 94/05**

(84) Benannte Vertragsstaaten:
**DE FR**

(56) Entgegenhaltungen:
**EP-A- 193 347**
**EP-A- 0 002 615**
**EP-A- 0 008 184**
**EP-A- 0 074 758**
**US-A- 4 199 569**

(73) Patentinhaber: **CIBA-GEIGY AG**
**Klybeckstrasse 141**
**CH-4002 Basel(CH)**

(72) Erfinder: **Frei, Bruno, Dr.**
**Bündtenstrasse 16**
**CH-4410 Liestal(CH)**
Erfinder: **O'Sullivan, Anthony Cornelius, Dr.**
**Habsburgerstrasse 38**
**CH-4055 Basel(CH)**

(74) Vertreter: **Zumstein, Fritz, Dr. et al**
**Patentanwälte,**
**Dr. F. Zumstein,**
**Dipl.-Ing. F. Klingseisen,**
**Bräuhausstrasse 4**
**D-80331 München (DE)**

EP 0 184 173 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue 13β-Milbemycinderivate der nachstehenden Formel I, deren Herstellung sowie deren Verwendung zur Bekämpfung von Schädlingen wie Ekto- und Endoparasiten.

Parasitizide Milbemycin-derivate werden in den Druckschriften US-A-4 199 569, EP-A-8 184 und EP-A-2 615 bereits beschrieben.

Bei den erfindungsgemässen Verbindungen handelt es sich um 13β-Milbemycine der allgemeinen Formel I

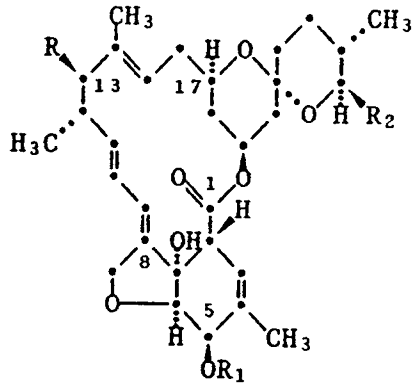

(I)

worin

- $R_1$ Wasserstoff oder eine übliche Schutzgruppe bedeutet;
- $R_2$ für Methyl, Ethyl, Isopropyl oder sek.Butyl steht; und
- R für einen über Sauerstoff oder Schwefel gebundenen Rest $R_3$, ausgewählt aus der Reihe $C_1$-$C_{10}$-Alkyl, $C_1$-$C_{10}$-Haloalkyl, $C_2$-$C_{10}$-Alkoxyalkyl, $C_3$-$C_{10}$-Alkoxyalkoxyalkyl, $C_2$-$C_{10}$-Alkenyl, $C_2$-$C_{10}$-Haloalkenyl, $C_2$-$C_{10}$-Alkinyl, $C_2$-$C_{10}$-Haloalkinyl, unsubstituiertes oder durch Halogen, $C_1$-$C_3$-Alkyl, $C_1$-$C_3$-Haloalkyl, $C_1$-$C_3$-Alkoxy, $C_1$-$C_3$-Haloalkoxy, Cyano und/oder Nitro substituiertes Phenyl und unsubstituiertes oder durch Halogen, $C_1$-$C_3$-Alkyl, $C_1$-$C_3$-Haloalkyl, $C_1$-$C_3$-Alkoxy, $C_1$-$C_3$-Haloalkoxy, Cyano und/oder Nitro substituiertes Benzyl steht, oder R eine der Gruppen -SH oder -S-C(O)$OR_4$ repräsentiert, wobei $R_4$ für $C_1$-$C_{10}$-Alkyl, $C_1$-$C_{10}$-Haloalkyl oder eine unsubstituierte oder durch Halogen, $C_1$-$C_3$-Alkyl, $C_1$-$C_3$-Haloalkyl, $C_1$-$C_3$-Alkoxy, $C_1$-$C_3$-Haloalkoxy, Cyano und/oder Nitro substituierte Gruppe aus der Reihe Phenyl und Benzyl steht.

Unter dem Begriff Alkyl selbst oder als Bestandteil eines anderen Substituenten sind je nach Zahl der angegebenen Kohlenstoffatome beispielsweise folgenden Gruppen zu verstehen: Methyl, Ethyl, Propyl, Butyl, Pentyl, Hexyl, Heptyl, Octyl, Nonyl, Decyl, usw. sowie die Isomeren, wie z.B. Isopropyl, Isobutyl, tert.-Butyl, Isopentyl usw.. Haloalkyl steht für einen einfach bis perhalogenierten Alkylsubstituenten, wie z.B. $CHCl_2$, $CHF_2$, $CH_2Cl$, $CCl_3$, $CH_2F$, $CH_2CH_2Cl$, $CHBr_2$ usw., vorzugsweise $CF_3$. Unter Halogen soll hier und im folgenden Fluor, Chlor, Brom oder Jod, vorzugsweise Fluor, Chlor oder Brom, verstanden werden. Haloalkoxy steht für einen über Sauerstoff gebundenen Haloalkylrest, der, wie weiter oben ausgeführt, halogeniert sein kann. Alkenyl steht für einen mindestens durch eine C = C-Doppelbindung charakterisierten aliphatischen Kohlenwasserstoffrest, wie z.B. für Vinyl, Propenyl-(1), Allyl, Butenyl-(1), Butenyl-(2), Butenyl-(3), usw.. Haloalkenyl bedeutet dementsprechend einen derartigen Alkenylrest, der ein- oder mehrfach halogeniert ist. Alkinyl steht für eine gerade oder verzweigte Kohlenstoffkette, die durch mindestens eine C≡C-Dreifachbindung charakterisiert ist. Typische Vertreter sind: Ethinyl, Propionyl-(1), Propargyl, Butinyl-(1), usw..$C_2$-$C_{10}$-Alkoxyalkyl steht für eine unverzweigte oder verzweigte, durch ein Sauerstoffatom unterbrochene $C_2$-$C_{10}$-Alkylgruppe, somit z.B. für $CH_2OCH_3$, $CH_2CH_2OCH_3$ , $CH_2CH(CH_3)OCH_3$, $CH_2OC_2H_5$, $CH_2OC_3H_7$-i, $CH_2CH_2CH_2$ $OCH_3$ usw. $C_3$-$C_{10}$-Alkoxyalkoxyalkyl steht für eine unverzweigte oder verzweigte $C_3$-$C_{10}$-Alkylgruppe, die an zwei Stellen durch jeweils ein Sauerstoffatom unterbrochen ist. Typische Beispiele sind: $CH_2OCH_2OCH_3$, $CH_2CH_2OCH_2OCH_3$, $CH_2OCH_2CH_2OCH_3$, $CH_2OCH_2OC_2H_5$, $CH(CH_3)$-$OCH_2OC_3H_7$-i usw. Unter OH-Schutzgruppen für den Substituenten $R_1$ sollen hier und im folgenden die in der organischen Chemie üblichen Schutzfunktionen verstanden werden. Dabei handelt es sich insbesondere um Acyl- und Silylgruppen. Geeignete Acylgruppen sind beispielsweise die Reste $R_4'$ -C(O)-, worin $R_4'$ die bei $R_4$ unter Formel I angegebenen Bedeutungen hat und vorzugsweise für $C_1$-$C_6$-Alkyl, $C_1$-$C_6$-Haloalkyl oder unsubstituiertes oder durch Halogen, $C_1$-$C_3$-Alkyl, $CF_3$ oder Nitro substituiertes Phenyl steht. Als

geeignete Silylgruppen für $R_1$ kommt der Rest $-Si(R_5)(R_6)(R_7)$ in Frage, wobei $R_5$, $R_6$ und $R_7$ vorzugsweise unabhängig voneinander für $C_1$-$C_4$-Alkyl, Benzyl oder Phenyl stehen und beispielsweise eine der Gruppen Trimethylsilyl, tris(tert.Butyl)silyl, Diphenyl-tert.butylsilyl, bis(Isopropyl)methylsilyl,Triphenylsilyl usw. und insbesondere tert.Butyl-dimethylsilyl bilden. Die 5-OH-Gruppe kann auch als Benzylether oder Methoxiethoximethylether vorliegen.

Verbindungen, worin $R_2$ sek.Butyl darstellt, sollen hier und im folgenden gleichfalls zu den Milbemycin-Derivaten gerechnet werden, obwohl sie nach der üblichen Systematik nicht darunter fallen, sondern gemäss US-PS 4.173.571 von Avermectin-Derivaten abgeleitet sind.

Verbindungen der Formel I, worin $R_1$ eine Schutzgruppe darstellt, lassen sich durch einfache, z.B. hydrolytische Abspaltung der Schutzfunktion in die hochaktiven freien 5-Hydroxi-derivate ($R_1$ = H) überführen und haben somit Zwischenprodukte-Charakter. Im übrigen wird der biologische Wert dieser Verbindungen durch die Schutzgruppe im Prinzip nicht gemindert.

Die Substituenten R in 13-Position bedeuten in natürlich vorkommenden Milbemycinen ($R_1$ = H; $R_2$ = $CH_3$, $C_2H_5$ oder iso$C_3H_7$) stets Wasserstoff. Bei Avermectinen dagegen steht in der 13-Position ein $\alpha$-L-Oleandrosyl-$\alpha$-L-oleandrose-Rest, der über Sauerstoff in $\underline{\alpha}$-Konfiguration mit dem Makrolid-Molekül verknüpft ist. Avermectine unterscheiden sich strukturell ausserdem durch eine 23-OH-Gruppe oder $\Delta^{22,23}$-Doppelbindung und in der Regel durch einen Substituenten $R_2$ = sek.$C_4H_9$ von den Milbemycinen. Durch Hydrolyse des Zucker-Restes der Avermectine gelangt man leicht zu den entsprechenden Avermectin-aglykonen, die eine allylische 13$\alpha$-Hydroxy-Gruppe besitzen. Bei den Avermectin-derivaten der vorliegenden Anmeldung liegt die $\Delta^{22,23}$-Doppelbindung stets in hydrierter Form vor.

Folgende Untergruppen von Verbindungen der Formel I sind auf Grund ihrer ausgeprägten parasitiziden und insektiziden Wirkung besonders bevorzugt:
Interessante Gruppen im Umfang der Formel I bilden:

Gruppe Ia: Verbindungen der Formel I, worin $R_1$ Wasserstoff bedeutet; $R_2$ für Methyl, Ethyl, Isopropyl oder sek.Butyl steht; R für einen über Sauerstoff oder Schwefel gebundenen Rest $R_3$, ausgewählt aus der Reihe $C_1$-$C_4$-Alkyl, $C_2$-$C_4$-Alkenyl, unsubstituiertes oder durch Fluor, Chlor, Brom, Methyl, $CF_3$, Methoxy, Cyano und/oder Nitro substituiertes Phenyl und unsubstituiertes oder durch Fluor, Chlor, Brom, Methyl, $CF_3$, Methoxy. Cyano und/oder Nitro substituiertes Benzyl steht, oder eine der Gruppen -SH oder -S-C(O)-$OR_4$ repräsentiert, wobei $R_4$ für $C_1$-$C_4$-Alkyl, $C_1$-$C_4$-Haloalkyl oder eine unsubstituierte oder durch Fluor, Chlor, Brom, Methyl, $CF_3$, Methoxy, Cyano und /oder Nitro substituierte Gruppe aus der Reihe Phenyl und Benzyl steht.

Gruppe Ib: Verbindungen der Formel I, worin $R_1$ Wasserstoff bedeutet; $R_2$ für Methyl, Ethyl, Isopropyl oder sek.Butyl steht; R für einen über Sauerstoff oder Schwefel gebundenen Rest $R_3$, ausgewählt aus der Reihe $C_1$-$C_4$-Alkyl und $C_2$-$C_4$-Alkenyl steht oder eine der Gruppen -SH oder -S-C(O)$OR_4$ repräsentiert, wobei $R_4$ für $C_1$-$C_4$-Alkyl, $C_1$-$C_4$-Haloalkyl oder unsubstituiertes oder durch Fluor, Chlor, Brom, Methyl, $CF_3$, Methoxy, Cyano und/oder Nitro substituiertes Phenyl steht.

Gruppe Ic: Verbindungen der Formel I, worin $R_1$ Wasserstoff bedeutet; $R_2$ für Methyl, Ethyl, Isopropyl oder sek.Butyl steht; R für einen über Sauerstoff oder Schwefel gebundenen Rest $R_3$, ausgewählt aus der Reihe $C_1$-$C_4$-Alkyl und $C_2$-$C_4$-Alkenyl steht oder eine der Gruppen -SH oder -S-C(O)$OR_4$ repräsentiert, wobei $R_4$ für $C_1$-$C_4$-Alkyl oder $C_1$-$C_4$-Haloalkyl steht.

Gruppe Id: Verbindungen der Formel I, worin $R_1$ Wasserstoff bedeutet; $R_2$ für Ethyl oder Isopropyl steht; R für einen über Sauerstoff oder Schwefel gebundenen Rest $R_3$, nämlich für $C_1$-$C_2$-Alkyl steht, oder eine der Gruppen -SH oder -S-C(O)$OR_4$ repräsentiert, wobei $R_4$ für $C_1$-$C_2$-Alkyl oder $C_1$-$C_2$-Haloalkyl steht.

Gruppe Ie: Verbindungen der Formel I, worin $R_1$ Wasserstoff bedeutet; $R_2$ für Ethyl oder Isopropyl steht; R für einen über Sauerstoff oder Schwefel gebundenen Rest $R_3$, nämlich für Methyl steht oder eine der Gruppen -SH oder -S-C(O)$OR_4$ repräsentiert, wobei $R_4$ für Methyl steht.

Gruppe If: Verbindungen der Formel I, worin $R_1$ Wasserstoff bedeutet; $R_2$ für Ethyl oder Isopropyl steht; R für einen über Sauerstoff oder Schwefel gebundenen Rest $R_3$, der für ein geradkettiges oder verzweigtes $C_1$-$C_4$-Alkyl und insbesondere für Methyl oder Ethyl steht.

Besonders bevorzugte 5-Hydroxi-derivate der Formel I sind z.B.
13$\beta$-Methoxi-milbemycin D,
13$\beta$-Ethoxy-milbemycin D,
13$\beta$-Phenylthio-milbemycin D,
13$\beta$-p-Chlorphenoxycarbonylthio-milbemycin D,
13$\beta$-Mercapto-milbemycin D,
13$\beta$-Methylthio-milbemycin D,
13$\beta$-tert.-Butylthio-milbemycin D,
13$\beta$-Methylthio-milbemycin $A_4$,

13β-tert.-Butylthio-milbemycin $A_4$,
13β-Methoxi-milbemycin $A_4$,
13β-Methoximethoxi-milbemycin $A_4$,
13β-Ethylthio-milbemycin $A_4$,
13β-Ethoxi-milbemycin $A_4$.

Bevorzugte an der 5-Hydroxygruppe mit einer Schutzfunktion versehene Verbindungen der Formel I sind z.B.
5-O-tert.Butyldimethylsilyl-13β-methoxi-milbemycin D,
5-O-tert.Butyldimethylsilyl-13β-ethoxi-milbemycin D,
5-O-tert.Butyldimethylsilyl-13β-mercapto-milbemycin D,
5-O-tert.Butyldimethylsilyl-13β-methylthio-milbemycin D.

Die vorliegende Erfindung betrifft nicht nur die Verbindungen der Formel I, sondern gleichermassen das neue Verfahren zu deren Herstellung. Es wurde nämlich überraschend gefunden, dass man mit geeigneten Ver(thio)etherungsmitteln die nachstehend definierten Allylalkohole der Formel II, worin die allylische OH-Gruppe sich in der 15-Position des Moleküls befindet, derart ver(thio)ethern kann, dass der einzuführende Substituentn R stereospezifisch die 13β-Position des Moleküls einnimmt und nur in untergeordnetem Masse Nebenprodukte liefert, die in 15-Position substituiert sind. Ferner wurde gefunden, dass Verbindungen der Formel II, die eine 13β-Hydroxigruppe enthalten, sich unter Beibehaltung der 13β-Orientierung durch übliche Veretherungsmethoden in die 13β-Ether überführen lassen. Das erfindungsgemässe Verfahren bildet somit die Möglichkeit, in der 13β-Position von Milbemycin- oder 13-Deoxi-22,23-dihydro-avermectin-aglykon-derivaten gezielt den unter Formel I definierten Substituenten R einzuführen und damit zu hochwirksamen Parasitiziden und Insektiziden zu gelangen, die überdies für weitere Derivatisierungen eingesetzt werden können.

Ein weiterer Gegenstand der vorliegenden Erfindung ist somit ein Verfahren zur Herstellung der Verbindungen der Formel I, das darin besteht, dass man einen Allylalkohol der Formel II

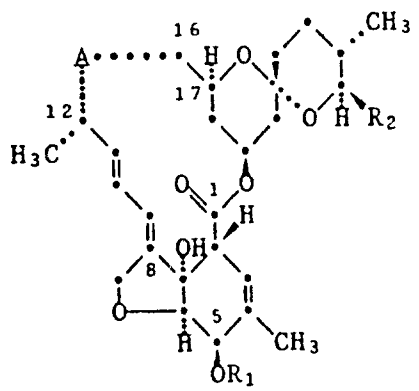

(II)

worin A für eine der Gruppen a oder b

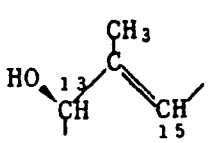 (a) oder 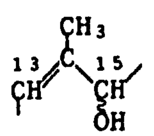 (b)

[= 13β-Hydroxi-$\Delta^{14,15}$] [= $\Delta^{13,14}$-15-Hydroxi]

steht, $R_1$ eine Schutzgruppe bedeutet und $R_2$ die unter Formel I angegebenen Bedeutungen hat, mit einem zur Einführung einer 13β-Ether- oder 13β-Thioethergruppe geeigneten Reagenz behandelt oder zwecks Einführung einer 13β-Mercaptogruppe mit einem Halothionoformiat behandelt und reduziert, woraufhin man die $R_1$-Schutzgruppe, sofern freie 5-Hydroxi-Verbindungen erwünscht sind, hydrolytisch abspaltet.

Allylalkohole der Formel II, worin A für die Gruppe a steht, sollen hier und im folgenden die Bezeichnung IIa besitzen, entsprechend sollen diejenigen, worin A für die Gruppe b steht, mit IIb bezeichnet werden.

Zur Einführung der 13β-Ether- oder 13β-Thioethergruppe in Verbindungen der Formel IIb geeignete Reagenzien sind beispielsweise

a) Alkohole und Thiole der Formel III

$R_3$-XH (III) ,

worin $R_3$ die unter Formel I angegebenen Bedeutungen hat und X für Sauerstoff oder Schwefel steht;

b) Halothionoformiate der Formel IV

$$Hal-\overset{\overset{S}{\|}}{C}-OR_4 \quad (IV),$$

worin $R_4$ die unter Formel I angegebenen Bedeutungen hat und Hal für Halogen, wie Fluor, Chlor, Brom oder Jod, vorzugsweise für Chlor oder Brom steht; sowie

c) Disulfide der Formel V

$R_3$-SS-$R_3$ (V) ,

worin $R_3$ die unter Formel I angegebenen Bedeutungen hat.

Die 13β-Alkohole der Formel IIa können auch nach üblichen Methoden, z.B. durch Umsetzung mit den Alkoholen der Formel III oder mit einem Halogenid $R_3$-Hal, worin $R_3$ die unter Formel I angegebenen Bedeutungen hat und Hal für ein Halogenatom, insbesondere Chlor oder Brom steht, in die 13β-Ether überführt werden. Man kann völlig analog auch ein zu den Alkoholen der Formel IIa analoges Thiol mit dem Halogenid $R_3$-Hal in 13β-Thioether überführen.Ebenso können Verbindungen der Formel I, worin R für eine 13β-Mercaptogruppe steht, auf übliche Art und Weise, z.B. durch Reaktion mit Alkylierungsmitteln der Formel III, in die 13β-Thioether überführt werden. Gemeint sind hierbei dem Fachmann geläufige Veretherungsreaktionen, die eine Derivatisierung einer 13β-Hydroxi-bzw. 13β-Mercapto-Gruppe darstellen und die räumliche 13β-Orientierung dieser Gruppen nicht antasten.

Das Verfahren wird im allgemeinen in einem reaktions-inerten Lösungsmittel oder in einem der beteiligten Reaktanden, sofern diese flüssig sind, durchgeführt. Geeignete Lösungsmittel sind z.B.: Ether und etherartige Verbindungen wie Dialkylether (Diethylether, Diisopropylether, tert.-Butylmethylether, Dimethoxyethan, Dioxan, Tetrahydrofuran, Anisol, usw.); halogenierte Kohlenwasserstoffe wie Chlorbenzol, Methylenchlorid, Ethylenchlorid, Chloroform, Tetrachlorkohlenstoff, Tetrachlorethylen, usw.; oder Sulfoxide wie Dimethylsulfoxid, wobei auch aromatische oder aliphatische Kohlenwasserstoffe, wie Benzol, Toluol, Xylole, Petrolether, Ligroin, Cyclohexan, usw. anwesend sein können. In manchen Fällen kann es von Vorteil sein, wenn die Reaktion oder Teilschritte davon unter Schutzgasatmosphäre (z.B. Argon, Helium, Stickstoff, etc.) und/oder in absoluten Lösungsmitteln durchgeführt werden. Gewünschtenfalls können Zwischenprodukte aus dem Reaktionsmedium isoliert und falls erwünscht, vor der Weiterreaktion, auf übliche Art und weise gereinigt werden, z.B. durch Waschen, Digerieren, Extraktion, Umkristallisation, Chromatographie usw. Mann kann jedoch auch auf derartige Reinigungsschritte verzichten und diese erst mit entsprechenden Endprodukten durchführen.

Die Umsetzung von Verbindungen der Formel II mit Alkoholen der Formel III bzw. von Verbindungen der Formel IIb mit Alkoholen oder Thiolen der Formel III erfolgt in Gegenwart katalytischer Mengen einer Säure. Als Säuren können hierbei Protonensäuren oder Lewis-Säuren eingesetzt werden. Beispiele geeigneter Säuren sind anorganische Säuren: Halogenwasserstoffsäure wie Chlorwasserstoffsäure, Bromwasserstoffsäure oder Jodwasserstoffsäure, Chlorsäure, Perchlorsäure sowie Schwefelsäure, Phosphorsäure, phosphorige Säure, und organische Säuren wie Essigsäure, Trifluoressigsäure, Trichloressigsäure, Propionsäure, Glykolsäure, Thiocyansäure, Milchsäure, Bernsteinsäure, Zitronensäure, Benzoesäure, Zimtsäure, Oxalsäure, Ameisensäure, Benzolsulfonsäure, p-Toluolsulfonsäure, Methansulfonsäure, Salicylsäure, p-Aminosalicylsäure, 2-Phenoxybenzoesäure, 2-Acetoxybenzoesäure, usw. sowie Lewis-Säuren, wie $BF_3$, $AlCl_3$, $ZnCl_2$ usw., insbesondere $BF_3$ als Etherat. Besonders bevorzugt sind Benzolsulfonsäure, P-Toluolsulfonsäure, Schwefelsäure und Bortrifluoridetherat. Bei dieser Reaktion kann es von Vorteil sein, wenn man zusätzlich in Gegenwart eines Orthoesters der Formel VI

$R_{10}C(OR_3)_3$ (VI) ,

worin $R_3$ wie unter Formel I definiert ist und $R_{10}$ für Wasserstoff oder $C_1$-$C_6$-Alkyl, vorzugsweise Methyl steht, arbeitet. Die Reaktionstemperaturen liegen im allgemeinen bei -50° bis +150°C, vorzugsweise bei -20° bis +100°C. bzw. am Siedepunkt des Lösungsmittels oder Lösungsmittelgemisches.

Die Reaktion von Verbindungen der Formel IIb mit Halothionoformiaten der Formel IV erfolgt üblicherweise in den oben genannten reaktionsinerten Lösungsmitteln oder in dem Halothionoformiat der Formel IV selbst. Man arbeitet zweckmässigerweise in Gegenwart eines Kondensationsmittels. Als solche kommen organische und anorganische Basen in Betracht, z.B. tertiäre Amine wie Trialkylamine (Trimethylamin, Triethylamin, Tripropylamin, usw.), Pyridin und Pyridinbasen (4-Dimethylaminopyridin, 4-Pyrrolidylaminopyridin usw.), bevorzugt ist Pyridin. Das Kondensationsmittel wird üblicherweise in mindestens äquimolarer Menge in Bezug auf die Ausgangsstoffe eingesetzt. Die Reaktionstemperaturen liegen bei dieser Umsetzung im allgemeinen bei -50° bis +150°C, vorzugsweise bei -20° bis +100°C. Die bei dieser Reaktion entstehenden Thiolcarbonate der Formel I (R = -S-C(O)$OR_4$) lassen sich durch einfache Reduktion, z.B. mit Zink in Eisessig, in die 13$\beta$-Mercapto-Verbindungen der Formel I (R=SH) überführen. Diese Reduktion erfolgt zweckmässigerweise in einem üblichen, reaktionsinerten, organischen Lösungsmittel bei Temperaturen zwischen 0° und 50°C, vorzugsweise 20° und 50°C.

Die Reaktion von Verbindungen der Formel IIb mit Disulfiden der Formel V erfolgt in Gegenwart einer mindestens äquimolaren Menge eines dreibindigen Phosphins, wie z.B. Triphenylphosphin, Tri-n-butylphosphin, n-Butyldiphenylphosphin und einer 1/10- bis 3-molaren Menge eines N-[$SR_3$]-Sulfenimids, worin $R_3$ die unter Formel I angegebenen Bedeutungen hat. Besonders geeignete Sulfenimide sind N-[$SR_3$]-Succinimid und N-[$SR_3$]-Benzosuccinimid. Die Reaktion wird zweckmässigerweise in einem reaktionsinerten Lösungsmittel bzw. Lösungsmittelgemisch durchgeführt. Geeignete Lösungsmittel sind die bereits weiter oben genannten Lösungsmittel. Man arbeitet in einem Temperaturbereich von 0° bis +50°C, vorzugsweise bei +20° bis +30°C.

Sofern nicht speziell aufgeführt, handelt es sich bei allen verwendeten Ausgangsprodukten um bekannte Verbindungen oder um Verbindungen, die sich in an sich bekannter Weise, z.B. analog zu den bekannten Vertretern, herstellen lassen.

Die Verbindungen der Formel IIb [= $\Delta^{13,14}$-15-Hydroxi] lassen sich aus 14,15-Epoxi-Milbemycinen der Formel VII gewinnen, worin $R_1$ und $R_2$ die für die Formel I genannten Bedeutungen haben,

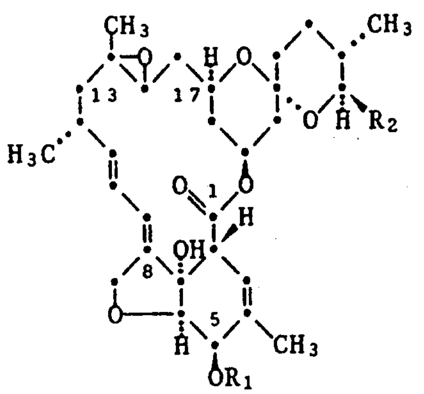

(VII)

mit Hilfe des Komplex-Reagenzes $[HN_3]_m/[Al(Ethyl)_3]_n$, worin m und n unabhängig voneinander die Zahl 1 oder 2 oder einen Zahlenwert zwischen 1 und 2 darstellen, in inerten trockenen Lösungsmitteln im Temperaturbereich von -30° bis +10°C, vorzugsweise -20° bis -5°C.

Als inerte Lösungsmittel kommen vorzugsweise aliphatische und aromatische Kohlenwasserstoffe wie Benzol, Toluol, Xylol, Petrolether; Ether wie Diethylether, tert.Butylmethylether, Tetrahydrofuran, Dioxan, Anisol in Frage.

Die Reaktion wird vorteilhaft unter Schutzgas, wie Stickstoff oder Argon, durchgeführt.

Stickstoffwasserstoffsäure $HN_3$ lässt sich in statu nascendi in den $[HN_3]_m/[Al(Et)_3]_n$-Komplex überführen, indem man im vorgesehenen trockenen Lösungsmittel oder Lösungsmittel-Gemisch Na-Azid suspendiert und daraus mit einer stärkeren Säure, z.B. $H_2SO_4$ (bevorzugt Oleum, um absolut trockene Reaktionsbedingungen zu gewährleisten), $HN_3$ in der Lösung in Freiheit setzt. $Al(Et)_3$ sollte in der Lösung bereits vorliegen oder kurz danach zugegeben werden. Die zur Reaktion vorgesehene Epoxi-Verbindung kann gleichfalls bereits vorliegen oder zu einem geeigneten Zeitpunkt zur Lösung dosiert werden.

Die zur Herstellung der Verbindungen IIb verwendeten Ausgangsverbindungen der Formel VII lassen sich leicht herstellen durch Epoxidierung der aus der US-PS 3,950,360 bekanntgewordenen und ursprünglich als "Antibiotika B-41-A", später als "Milbemycin-A"-Verbindungen und der aus der US-PS 4,346,171 bekanngewordenen und als "B-41-D" oder "Milbemycin-D" bezeichneten Verbindungen sowie der aus der US-PS 4,173,571 bekanntgewordenen 13-Deoxi-22,23-dihydro-Avermectine ($R_2$ = sec.Butyl) der nachstehenden Formel VIII

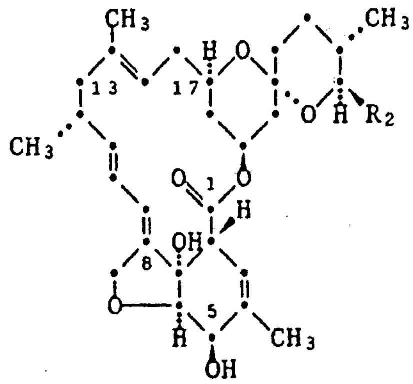

(VIII)

| | |
|---|---|
| $R_2 = CH_3$ | Milbemycin $A_3$ |
| $R_2 = C_2H_5$ | Milbemycin $A_4$ |
| $R_2 = isoC_3H_7$ | Milbemycin D |
| $R_2 = sec.C_4H_9$ | 13-Deoxi-22,23-dihydro-C-076-Bla-aglycon. |

Die Epoxidierung wird in einer Lösungsmittelphase im Temperaturbereich von -10° bis +20°C, vorzugsweise -5° bis +5°C, durchgeführt.

Die Epoxidierung wird mit Persäuren wie Peressigsäure, Trifluorperessigsäure, Perbenzoesäure, Chlorperbenzoesäure durchgeführt.

Die 13$\beta$-Hydroxi-$\Delta^{14,15}$-Verbindungen der Formel IIa lassen sich aus Verbindungen der Formel IIb, worin $R_1$ für eine Schutzgruppe steht, durch Reaktion mit Pyridiniumdichromat [= $(Pyr)_2{}^+Cr_2O_7$] herstellen. Man arbeitet hierbei in Dimethylformamid und bei Temperaturen zwischen ca. -10° und +60°C. Die $R_1$-Schutzgruppe wird, sofern gewünscht, anschliessend hydrolytisch abgespalten.

Durch Acylierung oder Silylierung der 5-OH-Gruppe werden alle jene Derivate der Formeln I, IIa, IIb und VII hergestellt, bei denen $R_1$ eine andere Bedeutung als Wasserstoff ($R_1$ = OH-Schutzgruppe) hat. Die Einführung der Acylgruppe erfolgt üblicherweise mit den entsprechenden Acylhalogeniden oder Acylanhydriden und wird vorzugsweise zur Einführung der eingangs definierten $R_4C(O)$- Gruppe benutzt. Zur Silylierung verwendet man zweckmässigerweise ein Silan der Formel Y-Si($R_5$)($R_6$)($R_7$), worin $R_5$, $R_6$ und $R_7$ einen der eingangs genannten Reste darstellen, wobei der Begriff Acylhalogenid für Acylchlorid oder Acylbromid steht und wobei Y eine Silylabgangsgruppe bedeutet. Zu den Silylabgangsgruppen Y zählen beispielsweise Bromid, Chlorid, Cyanid, Azid, Acetamid, Trifluoracetat, Trifluormethansulfonat. Diese Aufzählung stellt keine Limitierung dar, der Fachmann kennt weitere typische Silylabgangsgruppen.

5-O-Acylierungen und 5-O-Silylierungen werden in wasserfreiem Milieu, vorzugsweise in inerten Lösungsmitteln und besonders bevorzugt in aprotischen Lösungsmitteln durchgeführt. Die Reaktion läuft vorteilhaft im Temperaturbereich von 0° bis +80°C, bevorzugt bei +10° bis +40°C, ab. Vorzugsweise wird eine organische Base zugegeben. Es kommen als solche beispielsweise tertiäre Amine wie Triethylamin, Triethylendiamin, Triazol und bevorzugt Pyridin, Imidazol oder 1,8-Diazabicyclo[5.4.0]-undec-7-en (DBU) in Frage.

Die Entfernung dieser Silyl- und Aylreste $R_1$ in der 5-Position geschieht durch selektive milde Hydrolyse (→ $R_1$ = H) mit z.B. Arylsulfonsäure in alkoholischer Lösung oder nach einer anderen dem Fachmann geläufigen Methode.

Das beschriebene Verfahren zur Herstellung der Verbindungen der Formel I ist in allen Teilschritten ein Bestandteil vorliegender Erfindung.

Die Verbindungen der Formel I eignen sich ausgezeichnet zur Bekämpfung von Schädlingen an Tieren und Pflanzen, darunter tierparasitären Ekto-Parasiten. Zu letzteren zählen unter der Ordnung Acarina

insbesondere Schädlinge der Familien Ixodidae, Dermanyssidae, Sarcoptidae, Psoroptidae; die Ordnungen Mallophaga; Siphonaptera, Anoplura (z.B. Familie der Haemotopinidae); unter der Ordnung Diptera insbesondere Schädlinge der Familien Muscidae, Calliphoridae, Oestridae, Tabanidae, Hippoboscidae, Gastrophilidae.

Die Verbindungen I sind auch einsetzbar gegen Hygiene-Schädlinge, insbesondere der Ordnungen Diptera mit den Familien Sarcophagidae, Anophilidae, Culicidae; der Ordnung Orthoptera, der Ordnung Dictyoptera (z.B. Familie Blattidae) und der Ordnung Hymenoptera (z.B. Familie Formicidae).

Die Verbindungen I besitzen auch nachhaltige Wirksamkeit bei pflanzenparasitären Milben und Insekten. Bei Spinnmilben der Ordnung Acarina sind sie wirksam gegen Eier, Nymphen und Adulte von Tetranychidae (Tetranychus spp. und Panonychus spp.).

Hohe Aktivität besitzen sie bei den saugenden Insekten der Ordnung Homoptera, insbesondere gegen Schädlinge der Familien Aphididae, Delphacidae, Cicadellidae, Psyllidae, Coccidae, Diaspididae und Eriophydidae (z.B. die Rostmilbe auf Citrusfrüchten): Der Ordnungen Hemiptera; Heteroptera und Thysanoptera; sowie bei den pflanzenfressenden Insekten der Ordnungen Lepidoptera; Coleoptera; Diptera und Orthoptera.

Sie sind ebenfalls als Bodeninsektizid gegen Schädlinge im Erdboden geeignet.

Die Verbindungen der Formel I sind daher gegen alle Entwicklungsstadien saugender und fressender Insekten an Kulturen wie Getreide, Baumwolle, Reis, Mais, Soja, Kartoffeln, Gemüse, Früchten, Tabak, Hopfen, Citrus, Avocados und anderen wirksam.

Die Verbindungen der Formel I sind auch wirksam gegen Pflanzen-Nematoden der Arten Meloidogyne, Heterodera, Pratylenchus, Ditylenchus, Radopholus, Rizoglyphus und andere.

Besonders aber sind die Verbindungen gegen Helminthen wirksam, unter denen die endoparasitären Nematoden die Ursache schwerer Erkrankungen an Säugetieren und Geflügel sein können, z.B. an Schafen, Schweinen, Ziegen, Rindern, Pferden, Eseln, Hunden, Katzen, Meerschweinchen, Ziervögeln. Typische Nematoden dieser Indikation sind: Haemonchus, Trichostrongylus, Ostertagia, Nematodirus, Cooperia, Ascaris, Bunostonum, Oesophagostonum, Charbertia, Trichuris, Strongylus, Trichonema, Dictyocaulus, Capillaria, Heterakis, Toxocara, Ascaridia, Oxyuris, Ancylostoma, Uncinaria, Toxascaris und Parascaris. Der besondere Vorteil der Verbindungen der Formel I ist ihre Wirksamkeit gegen solche Parasiten, die gegen Wirkstoffe auf Benzimidazol-Basis resistent sind.

Gewisse Spezies der Arten Nematodirus, Cooperia und Oesophagostomum greifen den Intestinaltrakt des Wirtstiers an, während andere der Arten Haemonchus und Ostertagia im Magen und solche der Art Dictyocaulus im Lungengewebe parasitieren. Parasiten der Familien Filariidae und Setariidae finden sich im internen Zellgewebe und den Organen, z.B. dem Herzen, den Blutgefässen, den Lymphgefässen und dem subcutanen Gewebe. Hier ist vor allem der Herzwurm des Hundes, Dirofilaria immitis, zu nennen. Die Verbindungen der Formel I sind gegen diese Parasiten hoch wirksam.

Sie sind ferner zur Bekämpfung von humanpathogenen Parasiten geeignet, unter denen als typische, im Verdauungstrakt vorkommende Vertreter solche der Arten Ancylostoma, Necator, Ascaris, Strongyloides, Trichinella, Capillaria, Trichuris und Enterobius zu nennen sind. Wirksam sind die Verbindungen vorliegender Erfindung auch gegen Parasiten der Arten Wuchereria, Brugia, Onchocerca und Loa aus der Familie der Filariidae, die im Blut, im Gewebe und verschiedenen Organen vorkommen, ferner gegen Dracunculus und Parasiten der Arten Strongyloides und Trichinella, die speziell den Gastro-Intestinalkanal infizieren.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Verbindungen der Formel I werden bei Warmblütern in Aufwandmengen von 0,01 bis 10 mg/kg Körpergewicht angewendet, über geschlossenen Kultur-Anbauflächen, in Pferchen, Stallungen oder sonstigen Räumen in Mengen von 10 g bis 1000 g pro Hektar.

Die Formulierungen, d.h. die den Wirkstoff der Formel I enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie

Cyclohexanon, stark polare Lösungsmittel wie N-Methyl-2-pyrrolidon,Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Die in der Formulierungstechnik gebräuchlichen Tenside sind u.a. in folgender Publikation beschrieben:

"Mc Cutcheon's Detergents and Emulsifiers Annual"

MC Publishing Corp., Ridgewood, New Jersey, 1982.

Die pestiziden Zubereitungen enthalten in der Regel 0,01 bis 95 %, insbesondere 0,1 bis 80 %, Wirkstoff der Formel I, 5 bis 99,99 % eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel mit 1-10'000 ppm Wirkstoffgehalt.

Ein weiterer Gegenstand vorliegender Erfindung betrifft daher Schädlingsbekämpfungsmittel, die neben üblichen Trägerstoffen und/oder Verteilungsmitteln als mindestens einen Wirkstoff eine Verbindung der Formel I enthalten.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Herstellungsbeispiele

Herstellung von Ausgangs- und Zwischenprodukten

Beispiel A1: Herstellung von 14,15-Epoxi-Milbemycin D (Formel VII)

Zu einer Lösung von 550 mg Milbemycin D in 5 ml Dichlormethan wird unter Eiskühlung eine Lösung von 170 mg Chlorperbenzoesäure in 5 ml Dichlormethan gegeben. Nach 1-stündigem Rühren bei 0° bis +5°C werden nochmals 170 mg des Oxidationsmittels hinzugefügt und weitere 30 min gerührt. Nach beendeter Reaktion wird die Lösung in eine eisgekühlte Lösung von Natriumsulfit gegossen und mit Essigsäure-ethylester extrahiert. Die vereinigten Extrakte werden einmal mit Wasser gewaschen, getrocknet und im Vakuum eingedampft. Das Rohprodukt wird durch Chromatographierung über eine Silicagel-Säule

(Elutionsmittel n-Hexan/Essigsäureethylester 20:15) gereinigt. Es werden 450 mg 14,15-Epoxi-Milbemycin D als amorphe, weisse Substanz erhalten.

Beispiel A2: Herstellung von 15-Hydroxi-$\Delta^{13,14}$-Milbemycin D (Formel IIb)

Es werden bei -20 °C zu einer Lösung von 2,1 ml (1,75 g, 15,3 mmol) Triethylaluminium in 8,5 ml abs. Diethylether 9,5 ml (0,41 g, 9,53 mmol) einer 6,69 %igen Lösung von $HN_3$ in Diethylether gegeben, die dann bei -10 °C unter stark exothermer Reaktion zu 1,8 g (3,15 mmol) 14,15-Epoxi-Milbemycin D (in Substanz) gegeben wird. Nach 1 Stunde bei Raumtemperatur werden 4 ml absoluter Ether zugegeben und das gallertartige Reaktionsgemisch wird kräftig gerührt. Nach 4 Stunden wird wie in Vorschrift A1 aufgearbeitet und die Chromatographie an 70 g Kieselgel ($CH_2Cl_2$/Aceton 10:1) ergibt 200 mg (10 %) 14-Azido-15-hydroxi-Milbemycin D und 820 g (45 %) 15-Hydroxy-$\Delta^{13,14}$-Milbemycin D, Smp.: 151-153 °C (aus Methanol).

Beispiel A3: Herstellung von 5-O-t-Butyldimethylsilyl-14,15-epoxi-Milbemycin D (Formel VII)

Eine Lösung von 2,21 g (3,86 mmol) 14,15-Epoxi-Milbemycin D, 757 mg (5,02 mmol) t-Butyldimethylchlorsilan und 342 mg (5,02 mmol) Imidazol in 4 ml DMF wird 90 min bei Raumtemperatur gerührt. Anschliessend werden 80 ml Diethylether zugegeben, und das Gemisch wird über 20 g Kieselgel filtriert und eingeengt. Es werden 2,65 g (100 %) 5-O-t-Butyldimethylsilyl-14,15-epoxi-Milbemycin D erhalten. $^1$H-NMR (300 MHz. Lösungsmittel $CDCl_3$. Messwerte $\delta$ bezogen auf
$Si(CH_3)_4$ = TMS).
0,12 ppm (s) $(CH_3)_2Si$-O-;
0,92 ppm (s) (t.-$C_4H_9$)Si-O-;
1,23 ppm (breites s) $C_{14}CH_3$, d.h. Signal der $CH_3$-Gruppe in 14-Position);
2,56 ppm (d; J = 9) ($C_{15}H$, d.h. Signal des Protons in 15-Position).

Aehnlich lässt sich durch Reaktion mit Trimethylsilyl-trifluor-methansulfonat das entsprechende 5-O-Trimethylsilyl-14,15-epoxi-Milbemycin D herstellen, Smp. 92-97 °C.

Beispiel A4: Herstellung von 5-O-t-Butyldimethylsilyl-15-hydroxi-$\Delta^{13,14}$ -Milbemycin D (Formel IIb)

Eine Lösung des $HN_3/Et_3Al$-Komplex-Reagenz (hergestellt aus einer Lösung von 4,97 ml Triethylaluminium in 7 ml abs. Tetrahydrofuran (THF) und 9,15 ml einer 2,39 M Lösung von $HN_3$ (21,9 mmol) in abs. Diethylether) wird unter Argon zu einer Lösung von 5,0 g (7,29 mmol) 5-O-Butyldimethylsilyl-14,15-epoxi-Milbemycin D in ca. 20 ml abs. THF gegeben, und das Gemisch wird 15 Stunden unter Rückfluss erhitzt. Anschliessend werden bei Raumtemperatur 250 ml Ether, 2 ml Methanol und schliesslich ein Gemisch von 10 g $Na_2SO_4 \cdot 10\ H_2O$ und 10 g Celite zugegeben. Das Gemisch wird filtriert, eingeengt, und die Chromatographie des Rohproduktes an 160 g Kieselgel (O bis 30 % Essigsäureethylester in Hexan) ergibt 2,37 g (47 %) 5-O-t-Butyldimethylsilyl-15-hydroxi-$\Delta^{13,14}$-Milbemycin D.
$^1$H-NMR (300 MHz, $CDCl_3$):
1,59 ppm(d; J = 1), ($C_{14}CH_3$);
4,06 ppm (dd; $J_1$ = 11; $J_2$ = 4) ($C_{15}H$);
5,15 ppm (d; J = 8) ($C_{13}H$):
Daneben werden 109 mg (2 %) 13$\beta$-Azido-5-O-t-butyldimethylsilyl-Milbemycin D gewonnen.

Beispiel A5: Herstellung von 14,15-Epoxi-Milbemycin $A_4$ ($R_2 = C_2H_5$) [Formel VII]

Zu einer Lösung von 5,7 g (10,5 mmol) Milbemycin $A_4$ in 140 ml Dichlormethan und 120 ml 0,5 M $NaHCO_3$-Lösung wird bei Raumtemperatur eine Lösung von 2,43 g (14,08 mmol) m-Chlorobenzopersäure in 70 ml Dichlormethan tropfenweise zugegeben. Das Gemisch wird 1 Stunde bei Raumtemperatur kräftig gerührt und dann mit 300 ml Dichlormethan verdünnt. Die organische Phase wird mit wässriger $NaHCO_3$-Lösung gewaschen, mit $Na_2SO_4$ getrocknet und eingeengt. Es werden 5,7 g Epoxid als Rohprodukt erhalten.

Beispiel A6: Herstellung von 5-O-t-Butyldimethylsilyl-14,15-epoxi-Milbemycin $A_4$ (Formel VII)

5,7 g 14,15-Epoxi-Milbemycin $A_4$ werden in 10 ml trockenem Dimethylformamid (DMF) gelöst. Bei Raumtemperatur werden 0,63 g (9,16 mmol) Imidazol und 1,4 g (9,34 mmol) t-Butyldimethylchlorsilan

zugegeben. Das Gemisch wird 1 Stunde bei Raumtemperatur gerührt und an 150 g Kieselgel chromatographiert (Hexan/Ether 4:1), wobei 2,84 g (40 % d.Th. Ausbeute, bezogen auf Milbemycin $A_4$) des silylierten Epoxi-Derivats erhalten werden.

Beispiel A7: Herstellung von 5-O-t-Butyldimethylsilyl-15-hydroxi-$\Delta^{13,14}$-Milbemycin $A_4$ (Formel IIb)

Das Komplex-Reagenz $HN_3$/Al (Ethyl)$_3$ wird wie folgt hergestellt: 2,8 ml (12,2 mmol) $Al(C_2H_5)_3$ in 4 ml abs. THF werden unter Argon bei ca. -20 °C langsam mit 5,28 ml (20,4 mmol) einer 10 %igen Lösung von $HN_3$ in abs. Diethylether versetzt. Zu dieser Lösung wird unter Argon eine Lösung von 2,84 g (4,25 mmol) der im Beispiel A6 erhaltenen Verbindung gegeben, und das so erhaltene Gemisch wird 4 Stunden unter Rückfluss erhitzt. Bei Raumtemperatur werden 500 ml Diethylether, 10 g $Na_2SO_4 \cdot 10H_2O$ und 10 g Celite zugegeben, und das Gemisch wird filtriert und eingeengt. Die Chromatographie des Rohproduktes an 100 g Kieselgel (Hexan/Diethylether 7:2) ergibt 1,72 g (60 % d.Th.) der Titel-Verbindung.
$^1$H-NMR (300 MHz, $CDCl_3$):
1,59 ppm (br. s) ($C_{14}CH_3$);
4,05 ppm (br. s) ($C_{15}H$);
5,15 ppm (d; J = 6) ($C_{13}H$).

Daneben werden 0,1 g 13$\beta$-Azido-5-O-t-butyldimethylsilyl-Milbemycin $A_4$ erhalten.

Beispiel A8: Herstellung von 15-Hydroxi-$\Delta^{13,14}$-Milbemycin $A_4$ (Formel IIb)

Die Hydrolyse der im Beispiel A7 genannten Titelverbindung mit einer 1 %igen Lösung von p-Toluolsulfonsäure in Methanol und die Aufarbeitung in Diethylether mit 5 %iger Na-Hydrogencarbonat-Lösung ergibt die Titel-Verbindung.

Beispiel A9: Herstellung von 14,15-Epoxi-Milbemycin $A_3$($R_2 = CH_3$) (Formel VII)

Nach der Vorschrift des Beispiels A1 werden aus 220 mg Milbemycin $A_3$ in 5 ml Dichlormethan und 320 mg Benzoepersäure in 5 ml Dichlormethan bei -2° bis +5 °C während 1,5 Stunden und Reinigung über eine Silicagel-Säule 190 mg 14,15-Epoxi-Milbemycin $A_3$ gewonnen.

Beispiel A10: Herstellung von 5-O-tert.-Butyldimethylsilyl-14,15-epoxi-Milbemycin $A_3$ (Formel VII)

Nach der Vorschrift des Beispiels A3 werden aus 190 mg 14,15-Epoxi-Milbemycin $A_3$ und 120 mg tert.-Butyldimethylchlorsilan in Gegenwart von Imidazol 217 mg der Titel-Verbindung erhalten.

Beispiel A11: Herstellung von 5-O-tert.-Butyldimethylsilyl-15-hydroxi-$\Delta^{13,14}$-Milbemycin $A_3$ (Formel IIb)

Analog zur Epoxid-Spaltung des Beispiels A4 werden aus 210 mg 5-O-tert.-Butyldimethylsilyl-14,15-epoxi-Milbemycin $A_3$ in absolutem Diethylether mit Hilfe des Komplex-Reagenz $HN_3/Et_3Al$ unter Argon nach anschliessender Reinigung 203 mg der Titelverbindung erhalten.
$^1$H-NMR (300 HMz, $CDCl_3$):
1,58 ppm (br. s) ($C_{14}CH_3$);
4,05 ppm (br. s) ($C_{15}H$);
5,15 ppm (d; J = 6)($C_{13}H$).

Beispiel A12: Herstellung von 15-Hydroxi-$\Delta^{13,14}$-Milbemycin $A_3$ (Formel IIb)

Analog zu Beispiel A1 wird das Reagenz $HN_3/Al(C_2H_5)_3$ frisch hergestellt und bei -10 °C zu einer Lösung von 830 mg (3,05 mmol) 14,15-Epoxi-Milbemycin $A_3$ in 7 ml trockenem Diethylether getropft. Nach der Aufarbeitung werden 385 mg 15-Hydroxi-$\Delta^{13,14}$-Milbemycin $A_3$ und 92 mg 14-Azido-15-hydroxi-Milbemycin $A_3$ erhalten.

Beispiel A13: Herstellung von 13-Deoxi-14,15-epoxi-22,23-dihydro-avermectin-Bla-aglykon ($R_2$ = sec.$C_4H_9$) (Formel VII)

Analog zu Beispiel A5 erhält man aus 520 mg 13-Deoxi-22,23-dihydro-avermectin-Bla-aglykon [Tetrahedron Letters, Vol. 24, No. 148, pp. 5333-5336 (1983)] und 210 mg m-Chlorbenzopersäure in 20 ml

Dichlormethan 510 mg der Titelverbindung.

Beispiel A14: Herstellung von 5-O-t.Butyldimethylsilyl-13-deoxi-14,15-epoxi-22,23-dihydro-avermectin-Bla-aglykon (Formel VII)

Analog zu Beispiel A6 erhält man aus 220 mg der Titelverbindung von Beispiel A13 und 55 mg tert.Butyldimethylchlorsilan in Gegenwart von 25 mg Imidazol in 5 ml trockenem DMF 108 mg der Titelverbindung.

Beispiel A15: Herstellung von 13-Deoxi-15-hydroxi-$\Delta^{13,14}$-22,23-dihydro-avermectin-Bla-aglykon (Formel IIb)

Analog zu Beispiel A2 erhält man aus 220 mg der Titelverbindung von Beispiel A14 mit dem Komplex-Reagenz, bestehend aus 320 mg $Al(C_2H_5)_3$ und 110 mg einer 6,96 %igen Lösung von $HN_3$ in total 16 ml trockenem Diethylether 112 mg der Titelverbindung. Daneben werden 61 mg 13-Deoxi-14-azido-15-hydroxi-22,23-dihydro-avermectin-Bla-aglykonerhalten.

Beispiel A16: Herstellung von 5-O-tert.Butyldimethylsilyl-13β-hydroxi-milbemycin D und von 13β-Hydroxi-milbemycin D (Formel IIa)

Eine Lösung bestehend aus 286 mg (0,41 mmol) 5-O-tert.Butyldimethylsilyl-15-hydroxi-$\Delta^{13,14}$-milbemycin D und 209 mg (0,56 mmol) Pyridiniumdichromat (PDC) in 3 ml Dimethylformamid (DMF) wird 30 min bei Raumtemperatur gerührt. Anschliessend wird 1 ml Isopropanol zugegeben, 5 min weitergerührt und dann mit 50 ml Ether verdünnt. Nach weiteren 10 min wird das Gemisch durch Kieselgel filtriert und eingeengt. Bei der Chromatographie des Rohproduktes an 20 g Kieselgel (Ether/Hexan 1:2) werden 165 mg (57 %) 5-O-t-Butyldimethylsilyl-13β-hydroxi-Milbemycin D erhalten.
$^1$H-NMR (300 MHz; $CDCl_3$; TMS):
1,59 ppm (br. s)($C_{14}CH_3$)
3,70 ppm (d; J = 10)($C_{13}H$).

105 mg (0,153 mmol) der so gewonnenen Verbindung werden mit 1 ml einer 1 %igen Lösung von p-Toluolsulfonsäure in Methanol 1 Stunde bei Raumtemperatur gerührt. Das Gemisch wird mit 20 ml Ether verdünnt, durch Kieselgel filtriert, eingeengt, und der Rückstand wird an ca. 10 g Kieselgel chromatographiert (Aceton/Dichlormethan 1:4), wobei 73 mg (83 %) 13β-Hydroxi-Milbemycin D erhalten werden.
$^1$H-NMR (300 MHz; $CDCl_3$; TMS):
1,58 ppm (br.s)($C_{14}CH_3$)
3,71 ppm (d; J = 10)($C_{13}H$).

Herstellung von Endprodukten der Formel I

Beispiel H1: Herstellung von 13β-Methoxi-milbemycin D

Eine Lösung von 106 mg (0,155 mmol) 5-O-tert.Butyldimethylsilyl-15-hydroxi-$\Delta^{13,14}$-milbemycin D in 5 ml 1 %iger methanolischer Toluolsulfonsäure wird 4 Stunden unter Rückfluss erhitzt. Das Lösungsmittel wird abgedampft, der Rückstand in Diethylether aufgenommen und durch Kieselgel filtriert. Die Chromatographie des Rohproduktes (95 mg) [20 g Kieselgel/Laufmittel: Essigester/Hexan 2:3] ergibt 33 mg (36 %) 13β-Methoxi-milbemycin D mit folgenden spektroskopischen Daten:
$^1$H-NMR (300 MHz; $CDCl_3$; TMS):
1,48 ppm (s) ($C_{14}CH_3$)
1,87 ppm (s) $C_4CH_3$)
3,10 ppm (d; J = 9,8) $C_{13}H$)
3,15 ppm (s) $OCH_3$)
Massenspektrum m/e: 586 ($M^+$, 0,7 %, $C_{34}H_{50}O_7$), 568, 554, 514, 458, 426, 325, 307.

Beispiel H2: Herstellung von 5-O-tert.Butyldimethylsilyl-13β-methoxi-milbemycin D und 13β-Methoxi-milbemycin D

0,419 ml (406 mg; 3,83 mmol) Trimethylorthoformiat werden bei Raumtemperatur zu einer Lösung von 344 mg (0,501 mmol) 5-O-tert.-Butyldimethylsilyl-15-hydroxi-$\Delta^{13,14}$-milbemycin D in 3 ml einer 1 %igen Lösung von Schwefelsäure in Diethylether zutropfengelassen. Nach 10 min wird mit 5 %iger wässriger

$NaHCO_3$-Lösung und Diethylether aufgearbeitet. Die Chromatographie des Rohprodukts (327 mg) [20 g Kieselgel (Laufmittel:Aceton/Dichlormethan 1:100 (100 ml) und 1:50 (250 ml)] ergibt 107 mg (31 %) 5-O-tert.Butyldimethylsilyl-13β-methoxi-milbemycin D, das mit 2 ml einer Lösung von 40 %igem wässrigem HF/Acetonitril (5:95) 1 Stunde bei Raumtemperatur gerührt und dann mit 5 %iger wässriger $NaHCO_3$-Lösung und Diethylether aufgearbeitet wird. Die Chromatographie des Rohproduktes (75 mg) [12 g Kieselgel (Laufmittel:Ethylacetat/Hexan 2:3)] ergibt 71 mg 13β-Methoxy-milbemycin D mit den in Beispiel H1 genannten spektroskopischen Daten.

Analog zu Beispiel H2 werden auch die Verbindungen aus den Beispielen H2a bis H2c hergestellt.

Beispiel H2a: 13β-Methoxi-milbemycin $A_4$

$^1$H-NMR (250 MHz, $CDCl_3$, TMS)
3,16 (s) ($CH_3O$)
3,10 (d, J = 10Hz)($C_{13}H$)
Massenspektrum (FD) m/e: 572 ($M^+$, $C_{33}H_{48}O_8$) (D = Field Desorption)

Beispiel H3: Herstellung von 5-O-tert.Butyldimethylsilyl-13β-ethoxi-milbemycin D und 13β-Ethoxi-milbemycin D und 15-Ethoxi-$\Delta^{13,14}$-milbemycin D

a) 0,2 ml (225 mg; 1,39 mmol) Triethylorthoacetat werden bei Raumtemperatur zu einer Lösung von 264 mg (0,385 mmol) 5-O-tert.-Butyldimethylsilyl-15-hydroxi-$\Delta^{13,14}$-milbemycin D in 0,5 ml einer 1 %igen Lösung von Schwefelsäure in Diisopropylether und 1 ml Diethylether zugetropft. Nach 2 min wird mit 5 %iger wässriger $NaHCO_3$ und Diethylether aufgearbeitet. Die Chromatographie des Rohproduktes (230 mg) [20 g Kieselgel/Laufmittel:Diethyleter/Hexan 16:84] ergibt 164 mg (61 %) 5-O-tert.Butyldimethylsilyl-13β-ethoxi-milbemycin D und 34 mg (13 %) 5-O-tert.Butyldimethylsilyl-15-ethoxy-$\Delta^{13,14}$-milbemycin D.
$^1$H-NMR (300 MHz; $CDCl_3$;TMS)
von 5-O-tert.Butyldimethyl-15-ethoxi-$\Delta^{13,14}$-milbemycin D:
1,50 ppm (s) ($C_{14}CH_3$)
1,78 ppm (s) ($C_4CH_3$)
3,56 ppm (dd, J = 4,3 und 11,1), ($C_{15}H$)
5,08 ppm (dd, J = 1,1 und 9,3), ($C_{13}H$).

b) 164 mg (0,230 mmol) des nach a) hergestellten 5-O-tert.Butyldimethylsilyl-13β-ethoxi-milbemycins werden mit einer 1 %igen Lösung von p-Toluonsulfonsäure in Methanol 1 Stunde bei Raumtemperatur behandelt. Die Aufarbeitung mit Diethylether und 5 %iger wässriger $NaHCO_3$ und Chromatographie [20 g Kieselgel/Laufmittel:Ethylacetat/Hexan 2:3] ergibt 136 mg (99 %) 13β-Ethoxi-milbemycin D mit folgenden spektroskopischen Daten:
$^1$H-NMR (300 MHz, $CDCl_3$, TMS)
1,49 ppm (s) ($C_{14}CH_3$)
1,87 ppm (s) ($C_4CH_3$)
3,21 ppm (d, J = 9,8), ($C_{13}H$)
3,29 ppm (AB-System, J = 9,5; $_A$ = 3,17, aufgespalten in q; J = 7,0;
$\delta_B$ = 3,40; aufgespalten in q; J = 7,0), ($O\underline{CH_2}CH_3$).

Beispiel H4: Herstellung von 13β-Phenylthio-milbemycin D

Zu einer Lösung von 162 g (0,236 mmol) 5-O-tert.Butyldimethylsilyl-15-hydroxi-$\Delta^{13,14}$-milbemycin D und 0,3 ml (323 mg, 2,93 mmol) Thiophenol in 0,3 ml Dichlormethan und 0,1 ml $H_2SO_4$/Disopropylether (1:9) werden bei Raumtemperatur unter Rühren 0,086 ml (77 mg, 0,472 mmol) Triethylorthoacetat zugetropft. Nach 2 min wird mit 5 %iger wässriger $NaHCO_3$-Lösung und Diethylether aufgearbeitet. Die Chromatographie des Rohprodukts [3 g Kieselgel/Laufmittel:Hexan (40 ml); Diethylether/Hexan 1:4 (25 ml); Diethylether/Hexan 2:3 (25 ml)] liefert 110 mg des Rohprodukts. Dieses wird mit 2 ml einer 1 %igen Lösung von p-Toluolsulfonsäure in Methanol 1 Stunde bei Raumtemperatur gerührt, mit 5 %iger wässriger $NaHCO_3$ Lösung und Diethylether aufgearbeitet und chromatographiert [20 g Kieselgel/Laufmittel:Dichlormethan/Aceton 9:1]. Man erhält 33 mg (21 %) 13β-Phenylthio-milbemycin D mit den nachfolgend aufgeführten spektroskopischen Daten sowie 9 mg (5 %) 13β-Ethoxi-milbemycin D.
$^1$H-NMR (300 MHz; CDCl3; TMS):
1,58 ppm (s) ($C_{14}CH_3$)
1,87 ppm (s) ($C_4CH_3$)

3,33 ppm (d; J = 11,0) ($C_{13}H$)
4,78 ppm (ddd; J = 1,1; 5,3 und 11,3) ($C_{15}H$)
7,2-7,4 ppm (m) (Phenyl).
Massenspektrum m/e: 664 ($M^+$, $C_{39}H_{52}O_7S$) 646, 555, 554, 537, 385, 293, 275, 210, 209.

Beispiel H5: Herstellung von 13β-Phenylthio-milbemycin D

Zu einer Lösung von 139 mg (0,203 mmol) 5-O-tert.Butyldimethylsilyl-15-hydroxi-$\Delta^{13,14}$-milbemycin D und 0,080 ml (86 mg; 0,782 mmol) Thiophenol in 5 ml Dichlormethan, wird bei -10 °C unter Rühren unter Argon 0,060 ml (68 mg; 0,478 mmol) Bortrifluorid-ethyletherat zugetropft. Nach 10 min wird mit Diethylether und 5 %iger wässriger $NaHCO_3$-Lösung aufgearbeitet. Die Chromatographie des Rohprodukts [20 g Kieselgel/Laufmittel:Ethylacetat/Hexan 1:9 (100 ml); 3:7 (250 ml)] ergibt 37 mg (27 %) 13β-Phenylthio-milbemycin D mit den unter Beispiel H4 genannten spektroskopischen Daten.

Analog zu Beispiel H5 werden auch folgende Verbindungen der Beispiele H5a bis H5h hergestellt:

Beispiel H5a: 13β-Ethylthio-milbemycin D

$^1H$-NMR (250 MHz, $CDCl_3$, TMS)
2,27 (q, J = 5Hz)($CH_2$ - S)
3,05 (d, J = 10Hz)($C_{13}H$)
Massenspektrum (FD) m/e: 616 ($M^+$, ($C_{35}H_{52}O_7S$)

Beispiel H5b: 13β-Isopropylthio-milbemycin D

$^1H$-NMR (250 MHz, $CDCl_3$, TMS)
2,55 (m) [$(CH_3)_2C\underline{H}$-S]
3,05 (d, J = 10Hz)($C_{13}H$)
Massenspektrum (FD) m/e: 630 ($M^+$, $C_{36}H_{54}O_7S$)

Beispiel H5c: 13β-tert.-Butylthio-milbemycin D

$^1H$-NMR (300 MHz, $CDCl_3$, TMS)
1,29 (s) (S-tert.-Butyl)
1,59 (s) ($C_{14}CH_3$)
1,87 (s) ($C_4CH_3$)
3,12 (d, J = 10Hz)($C_{13}H$)
Massenspektrum m/e: 644 ($M^+$, $C_{37}H_{56}O_7S$), 210, 209, 181, 151.

Beispiel H5d: 13β-tert.-Butylthio-milbemycin $A_4$

$^1H$-NMR (250 MHz, $CDCl_3$, TMS)
1,62 (s) (S-tert.-Butyl)
3,15 (d, J = 10Hz)($C_{13}H$)
Massenspektrum (FD) m/e: 630 ($M^+$, $C_{36}H_{54}O_7S$)

Beispiel H5e: 13β-(2'-Ethoxiethylthio)-milbemycin D

$^1H$-NMR (250 MHz, $CDCl_3$, TMS)
2,52 (m) ($CH_2$-S)
3,07 (d, J = 10Hz)($C_{13}$-H)
3,54 (m) ($C\underline{H}_2$-O-$C\underline{H}_2$)

Beispiel H5f: 13β-Ethylthio-milbemycin $A_4$

$^1H$-NMR (250 MHz, $CDCl_3$, TMS)
2,36 (m) ($CH_2$-S)
3,04 (d, J = 10Hz)($C_{13}$-H)
Massenspektrum (FD) m/e: 602 ($M^+$, $C_{34}H_{50}O_7S$)

Beispiel H6: Herstellung von 13β-p-Chlorphenoxicarbonylthio-milbemycin D und 5-O-tert.Butyldimethylsilyl-13β-p-chlorphenoxicarbonylthio-milbemycin D

a) Zu einer Lösung von 151 mg (0,220 mmol) 5-O-tert.Butyldimethylsilyl-15-hydroxi-$\Delta^{13,14}$-milbemycin D und 0,089 ml (87 mg; 1,10 mmol) Pyridin in 3 ml Dichlormethan werden unter Argon bei -10 °C unter Rühren 0,036 ml (50 mg; 0,242 mmol) p-Chlorphenylchlorthionoformiat zugetropft. Nach 100 min Rühren bei Raumtemperatur werden weitere 0,036 ml p-Chlorphenylchlorthionoformiat zugetropft und nach einer weiteren Stunde wird mit 5 %iger wässriger $NaHCO_3$-Lösung und Diethylether aufgearbeitet. Die Chromatographie des Rohprodukts [20 g Kieselgel] liefert 221 mg rohes 5-O-tert.Butyldimethylsilyl-13β-p-chlorphenoxicarbonylthio-milbemycin D.
b) 140 mg dieses nach a) hergestellten Rohproduktes werden mit 1 ml einer Lösung von 40 %igem wässrigem HF/Acetoniril (5:95) 1 Stunde bei Raumtemperatur gerührt. Die Aufarbeitung mit 5 %iger wässriger $NaHCO_3$-Lösung und Diethylether und Chromatographie [20 g Kieselgel/Laufmittel:Ethylacetat/Hexan (2:3)] ergibt 69 mg (67 %) 13β-p-Chlorphenoxicarbonylthio-milbemycin D mit folgenden spektroskopischen Daten:
$^1$H-NMR (300 MHz; $CDCl_3$; TMS)
1,87 ppm (s) ($C_4CH_3$)
3,83 ppm (d, J = 11,7), ($C_{13}H$)
7,0-7,4 ppm (m) (Phenyl)
Massenspektrum m/e: <u>742</u> ($M^+$, $C_{40}H_{51}O_9SCl$) 614, 555, 427, 277, 209, 181, 151.

Beispiel H7: Herstellung von 13β-Mercapto-milbemycin D und von 5-O-tert.Butyldimethylsilyl-13β-mercapto-milbemycin D

a) Zu einer Lösung von 209 mg (0,305 mmol) 5-O-tert.Butyldimethylsilyl-15-hydroxi-$\Delta^{13,14}$-milbemycin D und 0,012 ml (120 mg, 1,52 mmol) Pyridin in 3 ml Dichlormethan werden unter Argon bei -10 °C unter Rühren 0,1 ml (157 mg, 0,689 mmol) Trichlorethylchlorthionoformiat zugetropft. Nach 1 Stunde Rühren bei Raumtemperatur wird mit 5 %iger wässriger $NaHCO_3$-Lösung und Diethylether aufgearbeitet. Die Chromatographie des Rohprodukts [20 g Kiesel/Laufmittel:Ethylacetat/Hexan (1:4)] liefert 282 mg z.T. noch verunreinigtes 5-O-tert.Butyldimethylsilyl-13β-trichlorethoxicarbonylthio-milbemycin D. Eine Suspension von 320 mg (4,9 mmol) Zink-Pulver in einer Lösung von 227 mg dieses Rohproduktes in 0,5 ml Diethylether, 2 ml 90 %iger wässriger Essigsäure und 3 Tropfen HCl (1M) werden bei Raumtemperatur 16 Stunden unter Argon gerührt. Das Gemisch wird mit Diethylether verdünnt, durch Celite filtriert, mit $MgSO_4$ getrocknet und eingeengt. Die Chromatographie des Rohprodukts [20 g Kieselgel/Laufmittel:Ethylcetat/Hexan (12:88)] ergibt 72 mg (40 %) 5-O-tert.-Butyldimethylsilyl-13β-mercapto-milbemycin D.
b) Dieses gereinigte Produkt wird mit 2 ml einer 1 %igen Lösung von p-Toluolsulfonsäure in Methanol 2 Stunden bei Raumtemperatur gerührt. Nach der Aufarbeitung mit 5 %iger wässriger $NaHCO_3$-Lösung und Diethylether wird das Rohprodukt [20 g Kieselgel/Laufmittel:Ethylcetat/Hexan (2:3)] chromatographiert. Man erhält 54 mg (89 %) 13β-Mercapto-milbemycin D mit folgenden spektroskopischen Daten: $^1$-NMR (300 MHz; $CDCl_3$; TMS)
1,61 ppm (s) ($C_{14}CH_3$)
1,87 ppm (s) ($C_4CH_3$)
3,31 ppm (dd; J = 5,4 und 10,9), ($C_{13}H$)
Massenspektrum m/e: <u>588</u> ($M^+$, $C_{33}H_{48}O_7S$) 460, 309, 277, 209, 181.

Beispiel H8: Herstellung von 13β-Methylthio-milbemycin D

a) 422 mg (0,615 mmol) 5-O-tert.Butyldimethylsilyl-15-hydroxi-$\Delta^{13,14}$-milbemycin D, 178 mg (1,23 mmol) N-Methylthio-succinimid und 323 mg (1,23 mmol) Triphenylphosphin werden unter Argon unter Rühren bei Raumtemperatur in 3 ml Dimethyldisulfid gelöst. Nach 10 min werden 0,4 ml Methanol zugegeben und das Lösungsmittel abgedampft. Das Rohprodukt wird chromatographiert [20 g Kieselgel/Laufmittel:Ethylacetat/Hexan 1:9 (200 ml) und 2:3 (250 ml)]. Man erhält 223 mg (53 %) 5-O-tert.Butyldimethylsilyl-13β-methylthio-milbemycin D, und als Nebenprodukte 36 mg (9 %) 5-O-tert.Butyldimethylsilyl-13β-hydroxi-milbemycin D und 28 mg (7 %) 5-O-tert.Butyldimethylsilyl-15-succinimido-$\Delta^{13,14}$-milbemycin D.
b) 160 mg (0,223 mmol) des so erhaltenen 5-O-tert.Butyldimethylsilyl-13β-methylthio-milbemycins D werden mit 1 %iger p-Toluolsulfonsäure in Methanol 1 Stunde bei Raumtemperatur behandelt. Die

Aufarbeitung mit 5 %iger wässriger $NaHCO_3$-Lösung und Diethylether, und Chromatographie [20 g Kieselgel/Laufmittel:Ethylacetat/Hexan (2:3)] ergibt 119 mg (89 %) 13β-Methylthio-milbemycin D mit folgenden spektroskopischen Daten:
$^1$H-NMR (300 MHz; $CDCl_3$; TMS)
1,56 ppm (s) ($C_{14}CH_3$)
1,88 ppm (s) ($C_4CH_3$ und $SCH_3$)
2,90 ppm (d; J = 11,0) ($C_{13}H$)
Massenspektrum m/e: 602 ($M^+$ ; $C_{34}H_{50}O_7S$), 474, 325, 323, 275, 210, 209.

Analog zu Beispiel H8 kann auch die im nachfolgenden Beispiel H8a genannte Verbindung hergestellt werden:

Beispiel H8a: 13β-Methylthio-milbemycin $A_4$

$^1$H-NMR (250 MHz; $CDCl_3$; TMS)
1,88 (s) ($CH_3S$)
2,92 (d, J = 10Hz)($C_{13}H$)
Massenspektrum m/e 588 ($M^+$, $C_{33}H_{48}O_7S$), 570, 530, 523, 461, 460, 413, 311, 309.

Beispiel H9: Herstellung von 13β-(2'-Methoxiethoximethoxi)-milbemycin D

Zu einer Lösung von 150 mg (0,218 mmol) 5-tert.-Butyldimethylsilyl-13β-hydroximilbemycin D und 225 µl (170 mg, 1,312 mmol) N,N-Diisopropylethylamin in 0,5 ml Dichlormethan werden unter Rühren bei Raumtemperatur 75 µl (82 mg, 0,656 mmol) 2-Methoxiethoximethylchlorid gegeben. Nach 3 Tagen bei Raumtemperatur wird mit Diethylether und 5-%iger wässriger $NaHCO_3$-Lösung aufgearbeitet. Die Diethyletherschicht wird über Magnesiumsulfat getrocknet, filtriert und eingeengt. Das ölige Rohprodukt wird mit 2 ml einer Lösung von 40 %iger wässriger Fluss-Säure/Acetonitril (5:95) eine Stunde bei Raumtemperatur gerührt und dann erneut mit 5-%iger wässriger $NaHCO_3$-Lösung und Diethylether aufgearbeitet. Ausbeute: 125 mg 13β-(2'-Methoxiethoximethoxi)-milbemycin D.

$^1$H-NMR (250 MHz, $CDCl_3$, TMS)
3,38 (s) ($CH_3O$)
3,55 (m) ($OC\underline{H_2}CH_2O$)
4,62 (AB-sytem, $\delta_A$ = 4,56; $\delta_B$ = 4,68, J = 7Hz)($OC\underline{H_2}O$)
Massenspektrum (FD) m/e: 660 ($M^+$, $C_{37}H_{56}O_{11}$).

Beispiel 9Ha: 13β-Methoximethoxi-milbemycin $A_4$

Die Herstellung erfolgt analog Beispiel H9.
$^1$H-NMR (250 MHz, $CDCl_3$, TMS)
3,33 (s) ($CH_3O$)
3,63 (d, J = 10Hz)($C_{13}H$)
4,42 und 4,60 (2d, J = 7Hz)($OC\underline{H_2}O$)
Massenspektrum (FD) m/e: 602 ($M^+$, $C_{34}H_{50}O_9$).

Beispiel 9Hb: 13β-Isobutylthio-milbemycin $A_4$

Die Herstellung erfolgt analog Beispiel H9.
$^1$H-NMR (300 MHz; $CDCl_3$, TMS)
1,55 (m) [$(CH_3)_2C$-S]
3,05 (d, J = 10Hz)($C_{13}H$)
Massenspektrum (FD) m/e: 654 ($M^+$, $C_{35}H_{52}O_7$)
Analog zu den beschriebenen Arbeitsweisen werden auch die nachfolgend genannten Verbindungen der Formel I hergestellt:

Tabelle 1: Typische Vertreter von Verbindungen der Formel I, worin $R_1$ für Wasserstoff steht ($C_6H_5$ steht für eine Phenylgruppe)

| Verb. Nr. | $R_2$ | R |
|---|---|---|
| 1.1 | $CH_3$ | $OCH_3$ |
| 1.2 | $C_2H_5$ | $OCH_3$ |
| 1.3 | $C_3H_7$-iso | $OCH_3$ |
| 1.4 | $C_4H_9$-sek | $OCH_3$ |
| 1.5 | $CH_3$ | $SCH_3$ |
| 1.6 | $C_2H_5$ | $SCH_3$ |
| 1.7 | $C_3H_7$-iso | $SCH_3$ |
| 1.8 | $C_4H_9$-sek | $SCH_3$ |
| 1.9 | $CH_3$ | $OC_2H_5$ |
| 1.10 | $C_2H_5$ | $OC_2H_5$ |
| 1.11 | $C_3H_7$-iso | $OC_2H_5$ |
| 1.12 | $C_4H_9$-sek | $OC_2H_5$ |
| 1.13 | $CH_3$ | $SC_2H_5$ |
| 1.14 | $C_2H_5$ | $SC_2H_5$ |
| 1.15 | $C_3H_7$-iso | $SC_2H_5$ |
| 1.16 | $C_4H_9$-sek | $SC_2H_5$ |
| 1.17 | $CH_3$ | $OC_6H_5$ |
| 1.18 | $C_2H_5$ | $OC_6H_5$ |
| 1.19 | $C_3H_7$-iso | $OC_6H_5$ |
| 1.20 | $C_4H_9$-sek | $OC_6H_5$ |
| 1.21 | $CH_3$ | $SC_6H_5$ |
| 1.22 | $C_2H_5$ | $SC_6H_5$ |
| 1.23 | $C_3H_7$-i | $SC_6H_5$ |
| 1.24 | $C_4H_9$-sek | $SC_6H_5$ |
| 1.25 | $CH_3$ | 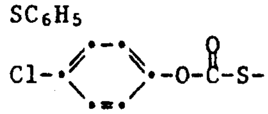 |
| 1.26 | $C_2H_5$ | 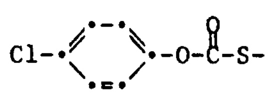 |
| 1.27 | $C_3H_7$-iso | 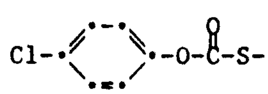 |

Tabelle 1: (Fortsetzung)

| Verb. Nr. | $R_2$ | R |
|---|---|---|
| 1.28 | $C_4H_9$-sek. | 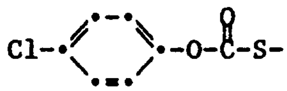 |
| 1.29 | $CH_3$ | SH |
| 1.30 | $C_2H_5$ | SH |
| 1.31 | $C_3H_7$-iso | SH |
| 1.32 | $C_4H_9$-sek | SH |
| 1.33 | $CH_3$ | $CCl_3CH_2-O-\overset{O}{\overset{\|}{C}}-S$ |
| 1.34 | $C_2H_5$ | $CCl_3CH_2-O-\overset{O}{\overset{\|}{C}}-S$ |
| 1.35 | $C_3H_7$-iso | $CCl_3CH_2-O\overset{O}{\overset{\|}{C}}-S$ |
| 1.36 | $C_4H_9$-sek | $CCl_3CH_2-O-\overset{O}{\overset{\|}{C}}-S$ |
| 1.37 | $C_3H_7$-iso | $SC_3H_7$-i |
| 1.38 | $C_3H_7$-iso | $SC_4H_9$-t |
| 1.39 | $C_3H_7$-iso | $OC_4H_9$-t |
| 1.40 | $C_3H_7$-iso | $OC_3H_7$-i |
| 1.41 | $C_2H_5$ | $SC_4H_9$-t |
| 1.42 | $C_2H_5$ | $OC_4H_9$-t |
| 1.43 | $C_3H_7$-iso | $SCH_2CH_2OC_2H_5$ |
| 1.46 | $C_3H_7$-iso | $SC_4H_9$-n |
| 1.49 | $C_3H_7$-iso | $OCH_2OCH_2CH_2OCH_3$ |
| 1.51 | $C_2H_5$ | $OCH_2OCH_3$ |
| 1.53 | $C_2H_5$ | $SC(CH_3)_2CH_2CH_3$ |
| 1.55 | $C_2H_5$ | $SC_4H_9$-n |
| 1.56 | $C_2H_5$ | $OC_4H_9$-n |

Tabelle 1: (Fortsetzung)

| Verb. Nr. | $R_2$ | R |
|---|---|---|
| 1.57 | $C_2H_5$ | $SCH_2C(CH_3)_3$ |
| 1.58 | $C_2H_5$ | $SCH_2CH_2C(CH_3)_3$ |
| 1.59 | $CH_3$ | $SCH_2C(CH_3)_3$ |
| 1.60 | $C_3H_7$-iso | $SCH_2C(CH_3)_3$ |
| 1.61 | $CH_3$ | $SCH_2CH_2C(CH_3)_3$ |

Diese Tabelle hat keinen limitierenden Charakter.

Formulierungsbeispiele für den Wirkstoff der Formel I (% = Gewichtsprozent)

| Spritspulver | a) | b) | c) |
|---|---|---|---|
| Wirkstoff Nr. 1.1 bis 1.61 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| Emulsions-Konzentrat | |
|---|---|
| Wirkstoff Nr. 1.1 bis 1.61 | 10 % |
| Octylphenolpolyethylenglykolether (4-5 Mol AeO) | 3 % |
| Ca-Dodecylbenzolsulfonat | 3 % |
| Ricinusölpolyglykolether (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| Stäubemittel | a) | b) |
|---|---|---|
| Wirkstoff Nr. 1.1 bis 1.61 | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

| Extruder Granulat | |
|---|---|
| Wirkstoff Nr. 1.1 bis 1.61 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| Tabletten bzw. Boli | | |
|---|---|---|
| I | Ein Wirkstoff 1.1 bis 1.61 | 33,0 % |
| | Methylcellulose | 0,80 % |
| | Kieselsäure hochdispers | 0,80 % |
| | Maisstärke | 8,40 % |

Methylcellulose in Wasser einrühren und quellen lassen; Kieselsäure in die Quellung einrühren und homogen suspendieren. Wirkstoff und Maisstärke mischen. In diese Mischung die wässrige Suspension einarbeiten und zu einem Teig kneten. Diese Masse durch ein Sieb (Maschenweite 12 M) granulieren und dann trocknen.

| | | |
|---|---|---|
| II | Milchzucker krist. | 22,50 % |
| | Maisstärke | 17,00 % |
| | mikrokrist. Cellulose | 16,50 % |
| | Magnesiumstearat | 1,00 % |
| Alle 4 Hilfsstoffe gut mischen | | |

Phasen I und II mischen und zu Tabletten oder Boli verpressen.

Falls die Verbindungen der Formel I bzw. entsprechende Mittel zur Bekämpfung von endoparasitären Nematoden, Cestoden und Trematoden bei Haus- und Nutztieren, wie Rindern, Schafen, Ziegen, Katzen und Hunden, verwendet werden, können sie den Tieren sowohl als Einzeldosis wie auch wiederholt verabreicht werden, wobei die einzelnen Gaben je nach Tierart vorzugsweise zwischen 0,1 und 10 mg pro kg Körpergewicht betragen. Durch eine protrahierte Verabreichung erzielt man in manchen Fällen eine bessere Wirkung oder man kann mit geringeren Gesamtdosen auskommen. Der Wirkstoff bzw. die ihn enthaltenden Mittel können auch dem Futter oder den Tränken zugesetzt werden. Das Fertigfutter enthält die Wirkstoffkombinationen vorzugsweise in einer Konzentration von 0,005 bis 0,1 Gew. %. Die Mittel können in Form von Lösungen, Emulsionen, Suspensionen, Pulver, Tabletten, Bolussen oder Kapseln peroral den Tieren verabreicht werden. Soweit die physikalischen und toxikologischen Eigenschaften von Lösungen oder Emulsionen dies zulassen, können die Verbindungen der Formel I bzw. die enthaltende Mittel an Tieren auch beispielsweise subcutan injiziert, intraruminal verabreicht oder mittels der Pour-on-Methode auf den Körper der Tiere appliziert werden. Ferner ist eine Verabreichung des Wirkstoffs an die Tiere auch durch Lecksteine (Salz) oder Molasse-Blöcke möglich.

## Biologische Beispiele

### B-1. Insektizide Frassgift-Wirkung bei Spodoptera littoralis

Eingetopfte Baumwollpflanzen im 5-Blatt-Stadium werden mit einer acetonisch/wässrigen Versuchslösung besprüht, die 3, 12,5 oder 50 ppm der zu prüfenden Verbindung enthält.

Nach dem Antrocknen des Belags werden die Pflanzen mit ca. 30 Larven ($L_1$-Stadium) von Spodoptera littoralis besetzt. Pro Versuchsverbindung und pro Test-Spezies verwendet man zwei Pflanzen. Der Versuch wird bei ca. 24 °C und 60 % relativer Luftfeuchtigkeit durchgeführt.

Auswertungen und Zwischenauswertungen auf moribunde Tiere, Wachstum und Larven und Frassschäden erfolgen nach 24 Stunden, 48 Stunden und 72 Stunden.

Die Verbindungen der Formeln I aus den Herstellungsbeispielen erzielten bereits bei einer Wirkstoffkonzentration von 6 ppm eine vollständige Abtötung nach 24 Stunden. Die Verbindungen Nr. 1.6, 1.38 und 1.41 erzielten diese Wirkung sogar noch bei 3ppm.

B-2. Wirkung gegen pflanzenschädigende Akariden OP-sensible Tetranychus urticae

Die Primärblätter von Bohnenpflanzen (Phaseolus vulgaris) werden 16 Stunden vor dem Versuch mit einem durch T. urticae infestierten, aus einer Massenzucht stammenden Blattstück belegt. Die so mit allen Milben-Stadien infestierten Pflanzen werden nach Entfernung des Blattstücks mit einer Versuchslösung bis zur Tropfnässe besprüht, die wahlweise 0,4 ppm oder 1,6 ppm der zu prüfenden Verbindung enthält. Die Temperatur in der Gewächshauskabine beträgt ca. 25 °C.

Nach sieben Tagen wird unter dem Binokular auf Prozentsatz mobiler Stadien (Adulte und Nymphen) und auf vorhandene Eier ausgewertet.

Verbindungen der Formel I wie z.B. Nr. 1.38 erzielte bereits bei einer Wirkstoffkonzentration von 0,4 ppm vollständige Abtötung.

B-3. Wirkung gegen $L_1$-Larven von Lucilia sericata

1 ml einer wässrigen Suspension der zu prüfenden Aktivsubstanz werden so mit 3 ml eines speziellen Larvenzuchtmediums bei ca. 50 °C vermischt, dass ein Homogenisat von wahlweise 250 ppm oder 125 ppm Wirkstoffgehalt entsteht. In jede Reagenzglas-Probe werden ca. 30 Lucilia-Larven ($L_1$) eingesetzt. Nach 4 Tagen wird die Mortalitätsrate bestimmt. Die Verbindungen aus den Herstellungsbeispielen zeigen bei 250 ppm eine Wirkung von 100 %, wobei die Verbindungen Nr. 1.2, 1.6, 1.31, 1.37, 1.38, 1.41, 1.43, 1.49 und 1.51 diese Wirkung auch noch bei der reduzierten Wirkstoffkonzentration von 100 ppm erzielten.

B-4. Akarizide Wirkung gegen Boophilus microplus (Biarra-Stamm

Auf einer PVC-Platte wird waagrecht ein Klebstreifen so befestigt, das darauf 10 mit Blut vollgesogene Zecken-Weibchen von Boophilus microplus (Biarra-Stamm) nebeneinander in einer Reihe mit dem Rücken aufgeklebt werden können. Jeder Zecke wird mit einer Injektionsnadel 1 µl einer Flüssigkeit injiziert, die eine 1:1-Mischung von Polyethylenglykol und Aceton darstellt und in der eine bestimmte Wirkstoffmenge von wahlweise 1, 0,1 oder 0,01 µl pro Zecke gelöst ist. Kontrolltiere erhalten eine wirkstofffreie Injektion. Nach der Behandlung werden die Tiere unter Normalbedingungen in einem Insektarium bei ca. 28 °C und 80 % relativer Luftfeuchtigkeit gehalten, bis die Eiablage erfolgt und die Larven aus den Eiern der Kontrolltiere geschlüpft sind.

Die Aktivität einer geprüften Substanz wird mit der $IR_{90}$ bestimmt, d.h. es wird jene Wirkstoffdosis ermittelt, bei der noch nach 30 Tagen 9 von 10 Zeckenweibchen (= 90 %) Eier ablegen, die nicht schlupffähig sind.

Die Verbindungen Nr. 1.3, 1.6, 1.7, 1.11, 1.23, 1.31, 1.37, 1.38, 1.41 und 1.49 erzielten eine $IR_{90}$ von 0,5 µg.

B-5. Versuch an mit Nematoden (Haemonchus concortus und Trichostrongylus colubriformis) infizierten Schafen

Der Wirkstoff wird als Suspension formuliert mit einer Magensonde oder durch Injektion in den Pansen eines Schafes gegeben, das mit Haemonchus concortus und Trychostrongylus colubriformis künstlich infiziert worden ist. Pro Dosis werden 1 bis 3 Tiere verwendet. Jedes Schaf wird nur einmal mit einer einzigen Dosis behandelt, und zwar wahlweise mit 1 mg oder 0,2 mg/kg Körpergewicht. Die Evaluierung erfolgt durch Vergleich der Anzahl der vor und nach Behandlung im Kot der Schafe ausgeschiedenen Wurmeier.

Gleichzeitig und gleichartig infizierte aber unbehandelte Schafe dienen als Kontrolle. Schafe, die mit einer der Verbindungen der Formeln I bei 1 mg/kg behandelt wurden, zeigten im Vergleich zu unbehandelten, aber infizierten Vergleichsgruppen keinen Nematodenbefall (= komplette Reduktion der Wurmeier im Kot). Die Verbindungen Nr. 1.3, 1.6, 1.7, 1.11, 1.15, 1.27, 1.31 und 1.37 bewirkten dies auch noch bei 0,2 mg/kg.

B-6. Kontaktwirkung auf Aphis craccivora

Erbsenkeimlinge, die mit sämtlichen Entwicklungsstadien der Laus infiziert sind, werden mit einer aus einem Emulsionskonzentrat hergestellten Wirkstofflösung besprüht, die wahlweise 50 ppm, 25 ppm oder 12,5 ppm Wirkstoff enthält. Nach 3 Tagen wird auf mehr als 80 % tote bzw. abgefallene Blattläuse ausgewertet. Nur bei dieser Wirkungshöhe wird ein Präparat als wirksam eingestuft.

Die Verbindungen Nr. 1.2, 1.6, 1.7, 1.37, 1.41 und andere erzielten bei einer Konzentration von 12,5 ppm eine vollständige Abtötung (= 100 %).

B-7. Larvizidwirkung gegen Aedes aegypti

Auf die Oberfläche von 150 ml Wasser, das sich in einem Behälter befindet, wird soviel einer 0,1 %igen acetonischen Lösung des Wirkstoffes pipettiert, dass Konzentrationen von wahlweise 10 ppm, 3,3 ppm und 1,6 ppm erhalten werden. Nach Verdunsten des Acetons wird der Behälter mit ca. 30-40 3 Tage alten Aedes-Larven beschickt. Nach 1, 2 und 5 Tagen wird die Mortalität geprüft.

Die Verbindungen aus den Herstellungsbeispielen wie z.B. Nr. 1.3, 1.11, 1.27, 1.37, 1.38 und 1.41 bewirkten in diesem Test bei einer Konzentration von 1,6 ppm bereits nach einem Tag eine vollständige Abtötung sämtlicher Larven.

**Patentansprüche**

**1.** Verbindungen der Formel I

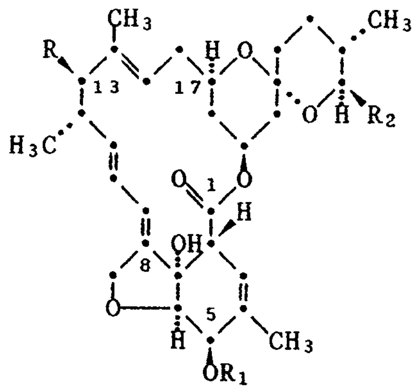

(I)

worin
$R_1$ Wasserstoff oder eine übliche Schutzgruppe bedeutet;
$R_2$ für Methyl, Ethyl, Isopropyl oder sek.Butyl steht; und R für einen über Sauerstoff oder Schwefel gebundenen Rest $R_3$, ausgewählt aus der Reihe $C_1$-$C_{10}$-Alkyl, $C_1$-$c_{10}$-Haloalkyl,$C_2$-$C_{10}$-Alkoxyalkyl, $C_3$-$C_{10}$-Alkoxyalkoxyalkyl, $C_2$-$C_{10}$-Alkenyl, $C_2$-$C_{10}$-Haloalkenyl, $C_2$-$C_{10}$-Alkinyl, $C_2$-$C_{10}$-Haloalkinyl, unsubstituiertes oder durch Halogen, $C_1$-$C_3$-Alkyl, $C_1$-$C_3$-Haloalkyl, $C_1$-$C_3$-Alkoxy, $C_1$-$C_3$-Haloalkoxy, Cyano und/oder Nitro substituiertes Phenyl und unsubstituiertes oder durch Halogen, $C_1$-$C_3$-Alkyl, $C_1$-$C_3$-Haloalkyl, $C_1$-$C_3$-Alkoxy, $C_1$-$C_3$-Haloalkoxy, Cyano und/oder Nitro substituiertes Benzyl steht, oder R eine der Gruppen -SH oder -S-C(O)$OR_4$ repräsentiert, wobei $R_4$ für $C_1$-$C_{10}$-Alkyl, $C_1$-$C_{10}$-Haloalkyl oder eine unsubstituierte oder durch Halogen, $C_1$-$C_3$-Alkyl, $C_1$-$C_3$-Haloalkyl, $C_1$-$C_3$-Alkoxy, $C_1$-$C_3$-Haloalkoxy, Cyano und/oder Nitro substituierte Gruppe aus der Reihe Phenyl und Benzyl steht.

**2.** Verbindungen der Formel I nach Anspruch 1, dadurch gekennzeichnet, dass $R_1$ für Wasserstoff oder eine der Gruppen $R_4$-C(O)-, oder -Si($R_5$)($R_6$)($R_7$) steht, wobei $R_5$, $R_6$, $R_7$ unabhängig voneinander für $C_1$-$C_4$-Alkyl, Benzyl oder Phenyl stehen und die Substituenten $R_2$, R, $R_3$ und $R_4$ wie unter Formel I definiert sind.

**3.** Verbindungen der Formel I nach Anspruch 2, worin $R_1$ Wasserstoff bedeutet; $R_2$ für Methyl, Ethyl, Isopropyl oder sek.Butyl steht; R für einen über Sauerstoff oder Schwefel gebundenen Rest $R_3$, ausgewählt aus der Reihe $C_1$-$C_4$-Alkyl, $C_2$-$C_4$-Alkenyl, unsubstituiertes oder durch Fluor, Chlor, Brom,

Methyl, $CF_3$, Methoxy, Cyano und/oder Nitro substituiertes Phenyl und unsubstituiertes oder durch Fluor, Chlor, Brom, Methyl, $CF_3$, Methoxy. Cyano und Nitro substituiertes Benzyl steht, oder eine der Gruppen -SH oder -S-C(O)$OR_4$ repräsentiert, wobei $R_4$ für $C_1$-$C_4$-Alkyl, $C_1$-$C_4$-Haloalkyl oder eine unsubstituierte oder durch Fluor, Chlor, Brom, Methyl, $CF_3$, Methoxy, Cyano und /oder Nitro substituierte Gruppe aus der Reihe Phenyl und Benzyl steht.

**4.** Verbindungen der Formel I nach Anspruch 3, worin $R_1$ Wasserstoff bedeutet; $R_2$ für Methyl, Ethyl, Isopropyl oder sek.Butyl steht; R für einen über Sauerstoff oder Schwefel gebundenen Rest $R_3$, ausgewählt aus der Reihe $C_1$-$C_4$-Alkyl und $C_2$-$C_4$-Alkenyl steht oder eine der Gruppen -SH oder -S-C-(O)$OR_4$ repräsentiert, wobei $R_4$ für $C_1$-$C_4$-Alkyl, $C_1$-$C_4$-Haloalkyl oder unsubstituiertes oder durch Fluor, Chlor, Brom, Methyl, $CF_3$, Methoxy, Cyano und/oder Nitro substituiertes Phenyl steht.

**5.** Verbindungen der Formel I nach Anspruch 4, worin $R_1$ Wasserstoff bedeutet; $R_2$ für Methyl, Ethyl, Isopropyl oder sek.Butyl steht; R für einen über Sauerstoff oder Schwefel gebundenen Rest $R_3$, ausgewählt aus der Reihe $C_1$-$C_4$-Alkyl und $C_2$-$C_4$-Alkenyl steht oder eine der Gruppen -SH oder -S-C-(O)$OR_4$ repräsentiert, wobei $R_4$ für $C_1$-$C_4$-Alkyl oder $C_1$-$C_4$-Haloalkyl steht.

**6.** Verbindungen der Formel I nach Anspruch 5, worin $R_1$ Wasserstoff bedeutet; $R_2$ für Ethyl oder Isopropyl steht; R für einen über Sauerstoff oder Schwefel gebundenen Rest $R_3$, nämlich für $C_1$-$C_2$-Alkyl steht, oder eine der Gruppen -SH oder -S-C(O)$OR_4$ repräsentiert, wobei $R_4$ für $C_1$-$C_2$-Alkyl oder $C_1$-$C_2$-Haloalkyl steht.

**7.** Verbindungen der Formel I nach Anspruch 6, worin $R_1$ Wasserstoff bedeutet; $R_2$ für Ethyl oder Isopropyl steht; R für einen über Sauerstoff oder Schwefel gebundenen Rest $R_3$, nämlich für Methyl steht oder eine der Gruppen -SH oder -S-C(O)$OR_4$ repräsentiert, wobei $R_4$ für Methyl steht.

**8.** Verbindungen der Formel I nach Anspruch 5, worin worin $R_1$ Wasserstoff bedeutet; $R_2$ für Ethyl oder Isopropyl steht; R für einen über Sauerstoff oder Schwefel gebundenen Rest $R_3$ aus der Reihe Methyl oder Ethyl steht.

**9.** Eine Verbindung der Formel I nach Anspruch 2, ausgewählt aus der Reihe
13β-Methoxi-milbemycin D,
13β-Ethoxy-milbemycin D,
13β-Phenylthio-milbemycin D,
13β-p-Chlorphenoxycarbonylthio-milbemycin D,
13β-Mercapto-milbemycin D,
13β-Methylthio-milbemycin D.

**10.** Eine Verbindung der Formel I nach Anspruch 2, ausgewählt aus der Reihe:
5-O-tert.Butyldimethylsilyl-13β-methoxi-milbemycin D,
5-O-tert.Butyldimethylsilyl-13β-ethoxi-milbemycin D,
5-O-tert.Butyldimethylsilyl-13β-mercapto-milbemycin D,
5-O-tert.Butyldimethylsilyl-13β-methylthio-milbemycin D.

**11.** Verfahren zur Herstellung einer Verbindung der Formel I nach Anspruch 1

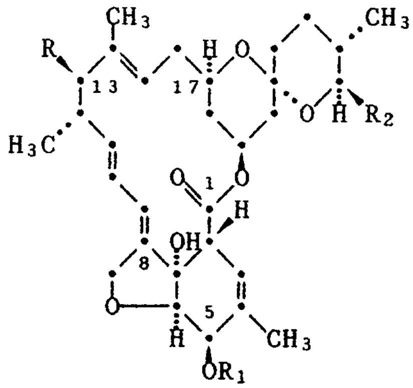

(I)

worin $R_1$, $R_2$ und R die Bedeutung gemäß Anspruch 1 besitzen, dadurch gekennzeichnet, dass man einen Allylalkohol der Formel II

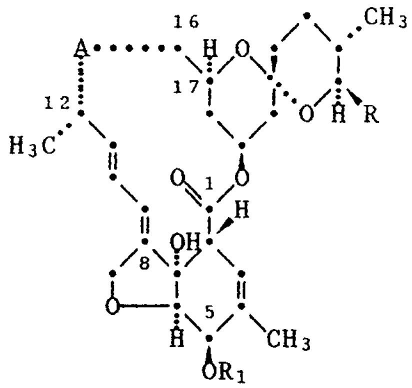

(II)

worin A für eine der Gruppen a oder b

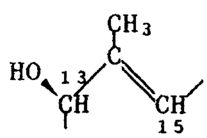

(a) oder

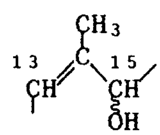

(b)

[= 13β-Hydroxi-$\Delta^{14,15}$] [= $\Delta^{13,14}$-15-Hydroxi]

steht, $R_1$ eine Schutzgruppe bedeutet und $R_2$ die unter Formel I angegebenen Bedeutungen hat, mit einem zur Einführung einer 13β-Ether- oder 13β-Thioethergruppe geeigneten Reagenz behandelt oder zwecks Einführung einer 13β-Mercaptogruppe mit einem Halothionoformiat behandelt und reduziert, woraufhin man die $R_1$-Schutzgruppe, sofern freie 5-Hydroxi-Verbindungen erwünscht sind, hydrolytisch abspaltet.

**12.** Verfahren nach Anspruch 11, dadurch gekennzeichnet, dass man als ein zur Einführung einer 13β-Ether- oder 13β-Thioethergruppe in eine Verbindung der Formel IIb geeignetes Reagenz einen Alkohol oder ein Thiol der Formel III

$R_3XH$ (III)

einsetzt, worin $R_3$ die unter Formel I angegebenen Bedeutungen hat und X für Sauerstoff oder Schwefel steht; oder als ein zur Einführung einer β-Thioethergruppe in eine Verbindung der Formel IIb

geeignetes Reagenz ein Halothionoformiat der Formel IV

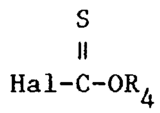

(IV)

einsetzt, worin $R_4$ die unter Formel I angegebenen Bedeutungen hat und Hal für Halogen steht, oder als ein zur Einführung einer $\beta$-Thioethergruppe in eine Verbindung der Formel IIb geeignetes Reagenz ein Disulfid der Formel V

$R_3$-SS-$R_3$ (V)

einsetzt, worin $R_3$ die unter Formel I angegebenen Bedeutungen hat.

**13.** Verfahren nach Anspruch 12, dadurch gekennzeichnet dass die Umsetzung mit Verbindungen der Formel III in Gegenwart einer katalytischen Menge einer Säure oder in Gegenwart einer katalytischen Menge einer Säure und zusätzlich in Gegenwart eines Orthoesters der Formel VI $R_6C(OR_3)_3$ (VI), worin $R_3$ die unter Formel I angegebenen Bedeutungen hat und $R_6$ für Wasserstoff oder $C_1$-$C_6$-Alkyl steht, bei Temperaturen zwischen -50° und +150°C, vorzugsweise -20° bis +100°C. stattfindet.

**14.** Verfahren nach Anspruch 12, dadurch gekennzeichnet, dass man die Umsetzung mit Verbindungen der Formel IV in einem reaktionsinerten Lösungsmittel oder dem Reagenz der Formel IV bei Temperaturen zwischen -50° und +150°C, vorzugsweise -20° bis +100°C, in Gegenwart einer Base durchführt.

**15.** Verfahren nach Anspruch 12 zur Herstellung von Verbindungen der Formel I, worin R für eine $\beta$-Mercaptogruppe steht, dadurch gekennzeichnet, dass man eine Verbindung der Formel I, worin R für die Gruppe -S-C(O)O$R_4$ steht, wobei $R_4$ die unter Formel I angegebenen Bedeutungen hat, bei Temperaturen zwischen 0° und 50°C zur 13$\beta$-Mercapto-Verbindung der Formel I reduziert.

**16.** Verfahren nach Anspruch 12, dadurch gekennzeichnet, dass man die Umsetzung mit einem Disulfid der Formel V in Gegenwart einer mindestens äquimolaren Menge eines dreibindigen Phosphins und einer 1/10- bis 3-molaren Menge eines N-[S$R_3$]-Sulfenimids, worin $R_3$ die unter Formel I angegebenen Bedeutungen hat, bei Temperaturen von 0° bis +50°C durchführt.

**17.** Verfahren zur Herstellung von 13$\beta$-Ether-derivaten der Formel I nach Anspruch 1

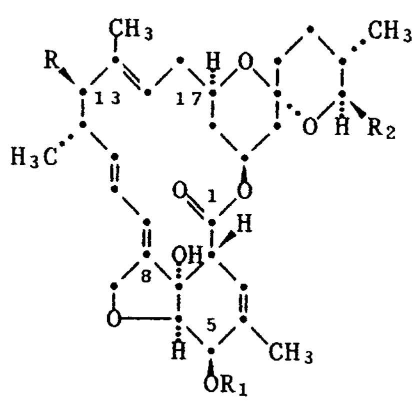

(I)

worin R für einen über Sauerstoff gebundenen Rest $R_3$ steht; und $R_1$, $R_2$ und $R_3$ die Bedeutung gemäß Anspruch 1 besitzen, dadurch gekennzeichnet, dass man einen 13$\beta$-Alkohol der Formel II

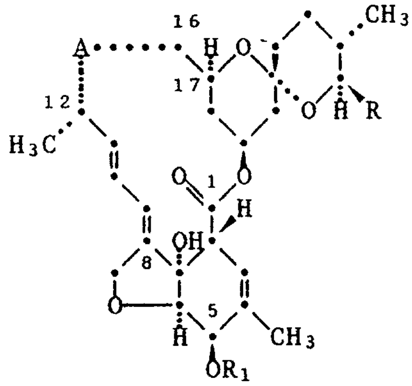

(II)

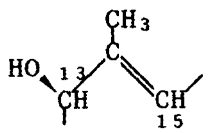

(a)

[= 13β-Hydroxi-$\Delta^{14,15}$]

steht, $R_1$ eine Schutzgruppe bedeutet; und $R_2$ die unter Formel I angegebenen Bedeutungen hat, durch übliche Veretherung, vorzugsweise mit einem Alkohol der Formel III

$R_3$-XH (III) ,

worin $R_3$ obige Bedeutungen hat und X für Sauerstoff steht, reagieren lässt.

**18.** Verfahren zur Herstellung von 13β-Thioether-derivaten der Formel I nach Anspruch 1

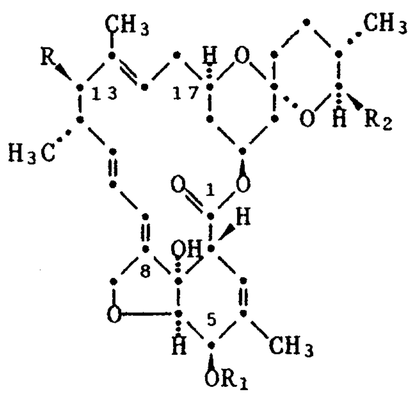

(I)

worin R für einen über Schwefel gebundenen Rest $R_3$ steht, oder R die Gruppe -S-C(O)$OR_4$ repräsentiert; und $R_1$, $R_2$, $R_3$ und $R_4$ die Bedeutung gemäß Anspruch 1 besitzen, dadurch gekennzeichnet, dass man ein 13β-Mercapto-derivat der Formel I, worin R für die 13β-Mercapto-Gruppe steht und die übrigen Substituenten wie oben definiert sind, auf übliche Weise verthioethert, vorzugsweise durch Reaktion mit einem Thiol der Formel III

$R_3$-XH (III) ,

worin $R_3$ obige Bedeutungen hat und X für Schwefel steht.

**19.** Schädlingebekämpfungsmittel gegen Ekto-, Endoparasiten und Insekten, dadurch gekennzeichnet, dass es neben üblichen Trägerstoffen und/oder Verteilungsmitteln mindestens eine Verbindung der Formel I gemäß Anspruch 1 enthält.

**20.** Mittel gemäss Anspruch 19, dadurch gekennzeichnet, dass es als Verbindung der Formel I mindestens eine Verbindung gemäss den Ansprüchen 2 bis 10 enthält.

**21.** Verfahren zur Bekämpfung von Schädlingen an Pflanzen, dadurch gekennzeichnet, dass man eine Verbindung der Formel I gemäss einem der Ansprüche 1 bis 10 auf die Pflanze oder deren Lebensraum appliziert.

**22.** Verfahren nach Anspruch 21, dadurch gekennzeichnet, dass es sich bei den Schädlingen um Insekten handelt.

**23.** Verbindung der Formel I nach einem der Ansprüche 1 bis 10 zur Verwendung in einem Verfahren zur Bekämpfung von Ekto- oder Endoparasiten beim Tier.

**24.** Verwendung einer Verbindung der Formel I nach einem der Ansprüche 1 bis 10 zur Herstellung eines Pharmazeutikums, vorzugsweise eines veterinärmedizinischen Pharmazeutikums.

## Claims

**1.** A compound of formula I

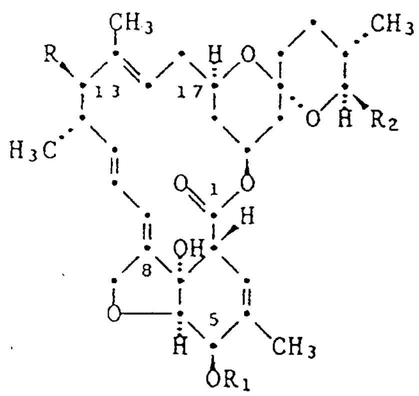

(I)

wherein

$R_1$ is hydrogen or a conventional protecting group;

$R_2$ is methyl, ethyl, isopropyl or sec-butyl; and

R is a radical $R_3$ which is bonded via oxygen or sulfur and is selected from the series comprising $C_1$-$C_{10}$alkyl, $C_1$-$C_{10}$haloalkyl, $C_2$-$C_{10}$alkoxyalkyl, $C_3$-$C_{10}$alkoxyalkoxyalkyl, $C_2$-$C_{10}$alkenyl, $C_2$-$C_{10}$haloalkenyl, $C_2$-$C_{10}$alkynyl, $C_2$-$C_{10}$haloalkynyl, phenyl which is unsubstituted or substituted by halogen, $C_1$-$C_3$alkyl, $C_1$-$C_3$haloalkyl, $C_1$-$C_3$alkoxy, $C_1$-$C_3$haloalkoxy, cyano and/or nitro and benzyl which is unsubstituted or substituted by halogen, $C_1$-$C_3$alkyl, $C_1$-$C_3$haloalkyl, $C_1$-$C_3$alkoxy, $C_1$-$C_3$haloalkoxy, cyano and/or nitro, or R is one of the groups -SH or -S-C(O)O$R_4$, where $R_4$ is $C_1$-$C_{10}$alkyl, $C_1$-$C_{10}$haloalkyl, or a group selected from the series comprising phenyl and benzyl which is unsubstituted or substituted by halogen, $C_1$-$C_3$alkyl, $C_1$-$C_3$haloalkyl, $C_1$-$C_3$alkoxy, $C_1$-$C_3$haloalkoxy, cyano and/or nitro.

**2.** A compound of formula I according to claim 1, wherein $R_1$ is hydrogen or one of the groups $R_4$-C(O)- or -Si($R_5$)($R_6$)($R_7$), where $R_5$, $R_6$ and $R_7$ independently of one another are $C_1$-$C_4$alkyl, benzyl or phenyl and the substituents $R_2$, R, $R_3$ and $R_4$ are as defined for formula I.

**3.** A compound of formula I according to claim 2, wherein $R_1$ is hydrogen; $R_2$ is methyl, ethyl, isopropyl or sec-butyl; and R is a radical $R_3$ which is bonded via oxygen or sulfur and is selected from the series comprising $C_1$-$C_4$alkyl, $C_2$-$C_4$alkenyl, phenyl which is unsubstituted or substituted by fluorine, chlorine, bromine, methyl, $CF_3$, methoxy, cyano and/or nitro, and benzyl which is unsubstituted or substituted by fluorine, chlorine, bromine, methyl, $CF_3$, methoxy, cyano and nitro, or R is one of the groups -SH or -S-C(O)O$R_4$, where $R_4$ is $C_1$-$C_4$alkyl, $C_1$-$C_4$haloalkyl, or a group from the series comprising phenyl and benzyl which is unsubstituted or substituted by fluorine, chlorine, bromine, methyl, $CF_3$, methoxy,

cyano and/or nitro.

**4.** A compound of formula I according to claim 3, wherein $R_1$ is hydrogen; $R_2$ is methyl, ethyl, isopropyl or sec-butyl; and R is a radical $R_3$ which is bonded via oxygen or sulfur and is selected from the series comprising $C_1$-$C_4$alkyl and $C_2$-$C_4$alkenyl, or R is one of the groups -SH or -S-C(O)$OR_4$, where $R_4$ is $C_1$-$C_4$alkyl, $C_1$-$C_4$haloalkyl, or phenyl which is unsubstituted or substituted by fluorine, chlorine, bromine, methyl, $CF_3$, methoxy, cyano and/or nitro.

**5.** A compound of formula I according to claim 4, wherein $R_1$ is hydrogen; $R_2$ is methyl, ethyl, isopropyl or sec-butyl; and R is a radical $R_3$ which is bonded via oxygen or sulfur and is selected from the series comprising $C_1$-$C_4$alkyl and $C_2$-$C_4$alkenyl, or R is one of the groups -SH or -S-C(O)$OR_4$, where $R_4$ is $C_1$-$C_4$alkyl or $C_1$-$C_4$haloalkyl.

**6.** A compound of formula I according to claim 5, wherein $R_1$ is hydrogen; $R_2$ is ethyl or isopropyl; and R is a radical $R_3$ which is bonded via oxygen or sulfur and is actually $C_1$-$C_2$alkyl, or R is one of the groups -SH or -S-C(O)$OR_4$, where $R_4$ is $C_1$-$C_2$alkyl or $C_1$-$C_2$haloalkyl.

**7.** A compound of formula I according to claim 6, wherein $R_1$ is hydrogen; $R_2$ is ethyl or isopropyl; and R is a radical $R_3$ which is bonded via oxygen or sulfur and is actually methyl, or R is one of the groups -SH or -S-C(O)$OR_4$, where $R_4$ is methyl.

**8.** A compound of formula I according to claim 5, wherein $R_1$ is hydrogen; $R_2$ is ethyl or isopropyl; and R is a radical $R_3$ which is bonded via oxygen or sulfur and is from the series comprising methyl and ethyl.

**9.** A compound of formula I according to claim 2, selected from the series comprising
13$\beta$-methoxymilbemycin D,
13$\beta$-ethoxymilbemycin D,
13$\beta$-phenylthiomilbemycin D,
13$\beta$-p-chlorophenoxycarbonylthiomilbemycin D,
13$\beta$-mercaptomilbemycin D and
13$\beta$-methylthiomilbemycin D.

**10.** A compound of formula I according to claim 2, selected from the series comprising
5-O-tert-butyldimethylsilyl-13$\beta$-methoxymilbemycin D,
5-O-tert-butyldimethylsilyl-13$\beta$-ethoxymilbemycin D,
5-O-tert-butyldimethylsilyl-13$\beta$-mercaptomilbemycin D and
5-O-tert-butyldimethylsilyl-13$\beta$-methylthiomilbemycin D.

**11.** A process for the preparation of a compound of formula I according to claim 1

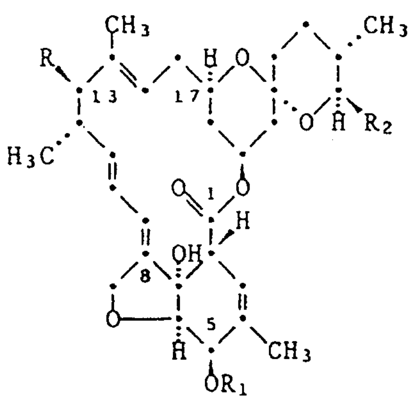

(I)

wherein $R_1$, $R_2$ and R are as defined in claim 1, which process comprises treating an allyl alcohol of formula II

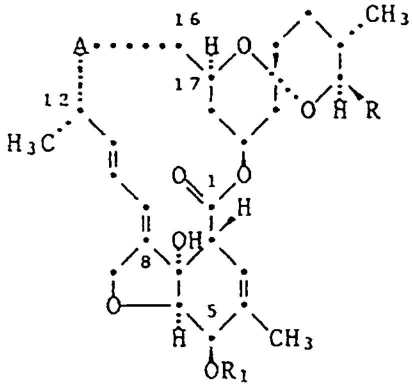

(II)

wherein A is one of the groups a or b

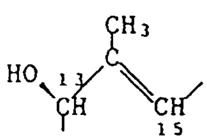

(a) or

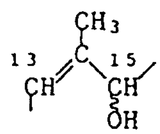

(b)

[= 13β-hydroxy-$\Delta^{14.15}$] [= $\Delta^{13.14}$-15-hydroxy]

$R_1$ is a protecting group and $R_2$ is as defined for formula I, with a reagent suitable for the introduction of a 13β-ether or 13β-thioether group or, to introduce a 13β-mercapto group, with a halothionoformate and then reducing the resultant product and, if a free 5-hydroxy compound is desired, subsequently removing the protecting group $R_1$ by hydrolysis.

**12.** A process according to claim 11 which comprises the use of an alcohol or a thiol of formula III

$R_3XH$ (III)

wherein $R_3$ is as defined for formula I and X is oxygen or sulfur, as a reagent suitable for the introduction of a 13β-ether or 13β-thioether group into a compound of formula IIb; or the use of a halothionoformate of formula IV

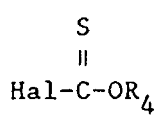

(IV)

wherein $R_4$ is as defined for formula I and Hal is halogen, as a reagent suitable for the introduction of a β-thioether group into a compound of formula IIb; or the use of a disulfide of formula V

$R_3$-SS-$R_3$ (V)

wherein $R_3$ is as defined for formula I, as a reagent suitable for the introduction of a β-thioether group into a compound of formula IIb.

**13.** A process according to claim 12, which comprises carrying out the reaction with a compound of formula III in the presence of a catalytic amount of an acid or in the presence of a catalytic amount of an acid and additionally in the presence of an orthoester of formula VI $R_6C(OR_3)_3$ (VI) wherein $R_3$ is as defined for formula I and $R_6$ is hydrogen or $C_1$-$C_6$ alkyl, at temperatures of between -50° and +150°C, preferably from -20° to +100°C.

**14.** A process according to claim 12, which comprises carrying out the reaction with a compound of formula IV in a reaction-inert solvent or in the reagent of formula IV at temperatures of between -50° and +150°C, preferably from -20° to +100°C, in the presence of a base.

**15.** A process according to claim 12 for the preparation of a compound of formula I, wherein R is a β-mercapto group, which process comprises reducing a compound of formula I, wherein R is the group $-S-C(O)OR_4$, $R_4$ being as defined for formula I, at temperatures of between 0° and 50°C to give the 13β-mercapto compound of formula I.

**16.** A process according to claim 12, which comprises carrying out the reaction with a disulfide of formula V in the presence of an at least equimolar amount of a trivalent phosphine and in the presence of a 1/10 to 3 molar amount of an N-[$SR_3$]-sulfenimide, wherein $R_3$ is as defined for formula I, at temperatures of from 0° to +50°C.

**17.** A process for the preparation of a 13β-ether derivative of formula I according to claim 1

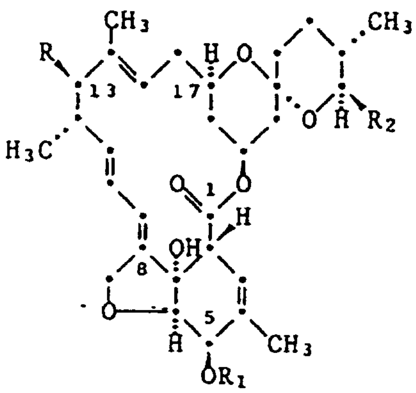

(I)

wherein

R is a radical $R_3$ which is bonded via oxygen, and $R_1$, $R_2$ and $R_3$ are as defined in claim 1, which process comprises reacting, by conventional etherification, a 13β-alcohol of formula II

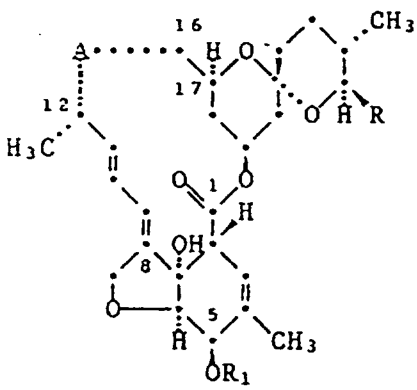

(II)

wherein A is the group a

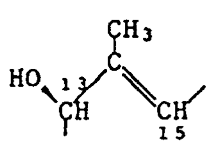

(a)

[= 13β-hydroxy-$\Delta^{14.15}$]

$R_1$ is a protecting group; and $R_2$ is as defined for formula I, preferably with an alcohol of formula (III)

$R_3$-XH (III)

in which $R_3$ is as defined above and X is oxygen.

**18.** A process for the preparation of a 13$\beta$-thioether derivative of formula I according to claim 1

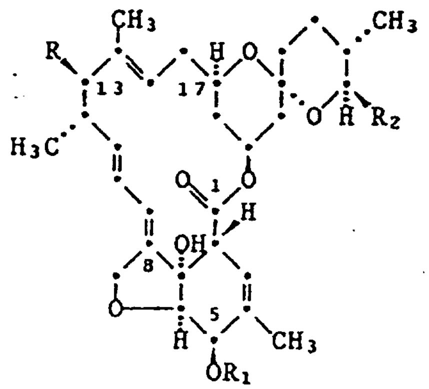

(I)

wherein
R is a radical $R_3$ which is bonded via sulfur, or R is the group -S-C(O)OR$_4$; and $R_1$, $R_2$, $R_3$ and $R_4$ are as defined in claim 1, which process comprises thioetherifying in conventional manner a 13$\beta$-mercapto derivative of formula I, wherein R is the 13$\beta$-mercapto group and the remaining substituents are as defined above, preferably by reaction with a thiol of formula III

$R_3$-XH (III)

wherein $R_3$ is as defined above and X is sulfur.

**19.** A pesticidal composition against ectoparasites, endoparasites and insects, which composition contains at least one compound of formula I according to claim 1 in addition to customary carriers and/or dispersing agents.

**20.** A composition according to claim 19, which contains as compound of formula I at least one compound according to claims 2 to 10.

**21.** A method of controlling pests of plants, which method comprises applying to the plant or to its habitat a compound of formula I according to any one of claims 1 to 10.

**22.** A method according to claim 21, wherein the pests are insects.

**23.** A compound of formula I according to any one of claims 1 to 10 for use in a method of controlling ectoparasites or endoparasites in or on animals.

**24.** The use of a compound of formula I according to any one of claims 1 to 10 for the preparation of a pharmaceutical, preferably a veterinary pharmaceutical.

## Revendications

1. Composés de formule I

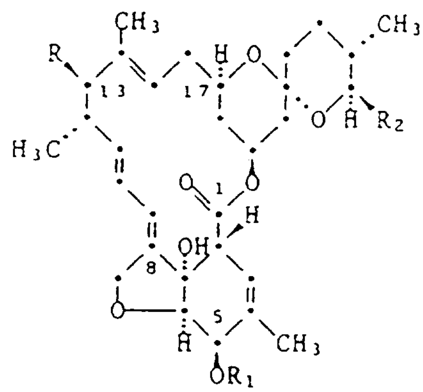

où
$R_1$ représente l'hydrogène ou un groupe protecteur usuel ; $R_2$ le méthyle, l'éthyle, l'isopropyle ou le sec-butyle ; et
R un reste $R_3$ lié par l'oxygène ou le soufre, choisi dans le groupe constitué par un alkyle en $C_1$-$C_{10}$, un haloalkyle en $C_1$-$C_{10}$, un alcoxyalkyle en $C_2$-$C_{10}$, un alcoxyalcoxyalkyle en $C_3$-$C_{10}$, un alcényle en $C_2$-$C_{10}$, un haloalcényle en $C_2$-$C_{10}$, un alcynyle en $C_2$-$C_{10}$, un haloalcynyle en $C_2$-$C_{10}$, un phényle non substitué ou substitué par un halogène, un alkyle en $C_1$-$C_3$, un haloalkyle en $C_1$-$C_3$, un alcoxy en $C_1$-$C_3$, un haloalcoxy en $C_1$-$C_3$, un cyano et/ou un nitro et un benzyle non substitué ou substitué par un halogène, un alkyle en $C_1$-$C_3$, un haloalkyle en $C_1$-$C_3$, un alcoxy en $C_1$-$C_3$, un haloalcoxy en $C_1$-$C_3$, un cyano et/ou un nitro ou R représente l'un des groupes -SH ou -S-C(O)$OR_4$, $R_4$ représentant un alkyle en $C_1$-$C_{10}$, un haloalkyle en $C_1$-$C_{10}$ ou un groupe constitué par le phényle ou le benzyle non substitué ou substitué par un halogène, un alkyle en $C_1$-$C_3$, un haloalkyle en $C_1$-$C_3$, un alcoxy en $C_1$-$C_3$, un haloalcoxy en $C_1$-$C_3$, le cyano et/ou le nitro.

2. Composés de formule I selon la revendication 1, caractérisés en ce que $R_1$ représente l'hydrogène ou l'un des groupes $R_4$-C(O)- ou -Si($R_5$)($R_6$)($R_7$), $R_5$, $R_6$, $R_7$ représentant, indépendamment les unes des autres, un alkyle en $C_1$-$C_4$, le benzyle ou le phényle et en ce que les substituants $R_2$, R, $R_3$ et $R_4$ sont définis comme pour la formule I.

3. Composés de formule I selon la revendication 2, où $R_1$ représente l'hydrogène ; $R_2$ représente le méthyle, l'éthyle, l'isopropyle ou le sec-butyle ; R représente un reste $R_3$ lié par l'oxygène ou le soufre, choisi dans le groupe constitué par un alkyle en $C_1$-$C_4$, un alcényle en $C_2$-$C_4$, un phényle non substitué ou substitué par le fluor, le chlore, le brome, le méthyle, $CF_3$, le méthoxy, le cyano et/ou le nitro et un benzyle non substitué ou substitué par le fluor, le chlore, le brome, le méthyle, $CF_3$, le méthoxy, le cyano et/ou le nitro ou représente l'un des groupes -SH ou -S-C(O)$OR_4$, $R_4$ représentant un alkyle en $C_1$-$C_4$, un haloalkyle en $C_1$-$C_4$ ou un groupe constitué par le phényle ou le benzyle non substitué ou substitué par le fluor, le chlore, le brome, le méthyle, $CF_3$, le méthoxy, le cyano et/ou le nitro.

4. Composés de formule I selon la revendication 3, où $R_1$ représente l'hydrogène ; $R_2$ représente le méthyle, l'éthyle, l'isopropyle ou le sec-butyle ; R représente un reste $R_3$ lié par l'oxygène ou le soufre, choisi dans le groupe constitué par un alkyle en $C_1$-$C_4$ et un alcényle en $C_2$-$C_4$ ou représente l'un des groupes -SH ou -S-C(O)$OR_4$, $R_4$ représentant un alkyle en $C_1$-$C_4$, un haloalkyle en $C_1$-$C_4$ ou un phényle non substitué ou substitué par le fluor, le chlore, le brome, le méthyle, $CF_3$, le méthoxy, le cyano et/ou le nitro.

5. Composés de formule I selon la revendication 4, où $R_1$ représente l'hydrogène ; $R_2$ représente le méthyle, l'éthyle, l'isopropyle ou le sec-butyle ; R représente un reste $R_3$ lié par l'oxygène ou le soufre, choisi dans le groupe constitué par un alkyle en $C_1$-$C_4$ et un alcényle en $C_2$-$C_4$ ou représente l'un des groupes -SH ou -S-C(O)$OR_4$, $R_4$ représentant un alkyle en $C_1$-$C_4$ ou un haloalkyle en $C_1$-$C_4$.

6. Composés de formule I selon la revendication 5, où $R_1$ représente l'hydrogène ; $R_2$ représente l'éthyle ou l'isopropyle ; R représente un reste, $R_3$ lié par l'oxygène ou par le soufre, plus particulièrement un alkyle en $C_1$-$C_2$ ou représente l'un des groupes -SH ou -S-C(O)$OR_4$, $R_4$ représentant un alkyle en $C_1$-$C_2$ ou un haloalkyle en $C_1$-$C_2$.

7. Composés de formule I selon la revendication 6, où $R_1$ représente l'hydrogène ; $R_2$ représente l'éthyle ou l'isopropyle ; R représente un reste $R_3$ lié par l'oxygène ou par le soufre, en particulier le méthyle ou représente l'un des groupes -SH ou -S-C(O)$OR_4$, $R_4$ représentant le méthyle.

8. Composés de formule I selon la revendication 5, où $R_1$ représente l'hydrogène ; $R_2$ l'éthyle ou l'isopropyle ; R représente un reste $R_3$ choisi dans le groupe constitué par le méthyle ou l'éthyle, lié par l'oxygène ou le soufre.

9. Composé de formule I selon la revendication 2, choisi dans le groupe :
13 $\beta$-méthoxy-milbémycine D,
13 $\beta$-éthoxy-milbémycine D,
13 $\beta$-phénylthio-milbémycine D,
13 $\beta$-p-chlorophénoxycarbonylthio-milbémycine D,
13 $\beta$-mercapto-milbémycine D,
13 $\beta$-méthylthio-milbémycine D.

10. Composé de formule I selon la revendication 2, choisi dans le groupe :
5-O-tert-butyldiméthylsilyl-13$\beta$-méthoxy-milbémycine D,
5-O-tert-butyldiméthylsilyl-13$\beta$-éthoxy-milbémycine D,
5-O-tert-butyldiméthylsilyl-13$\beta$-mercapto-milbémycine D,
5-O-tert-butyldiméthylsilyl-13$\beta$-méthylthio-milbémycine D.

11. Procédé pour la préparation d'un composé de formule I selon la revendication 1

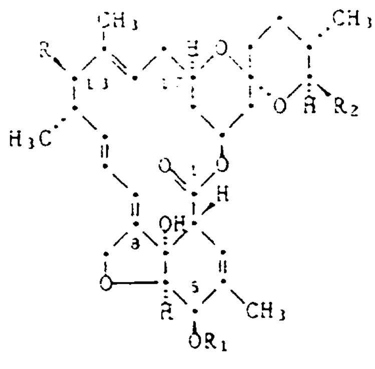

où $R_1$, $R_2$ et R ont la signification selon la revendication 1, caractérisé en ce que l'on traite un alcool allylique de formule II

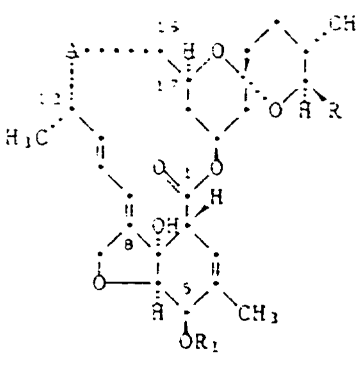

où A représente l'un des groupes a ou b

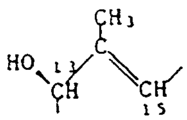 (a) ou 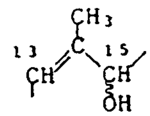 (b)

[= 13β-Hydroxy-$\Delta^{14,15}$] [= $\Delta^{13,14}$-15-Hydroxy ]

$R_1$ représente un groupe protecteur et $R_2$ a les 2 significations données pour la formule I, avec un réactif approprié pour l'introduction d'un groupe 13. β-éther ou 13 β-thioéther ou avec un halothionoformiate dans le but d'introduire un groupe 13 β-mercapto et en ce que l'on réduit, puis en ce que l'on clive par hydrolyse le groupe protecteur $R_1$, si l'on désire des composés 5-hydroxy libres.

**12.** Procédé selon la revendication 11, caractérisé en ce que l'on utilise, comme réactif approprié pour introduire un groupe 13 β-éther ou 13 β-thioéther dans un composé de formule IIb, un alcool ou un thiol de formule III

$R_3XH$ (III)

où $R_3$ a la signification donnée pour la formule I et X représente l'oxygène ou le soufre ; ou en ce que l'on utilise, comme réactif approprié pour introduire un groupe β-thioéther dans un composé de formule IIb, un halothionoformiate de formule IV

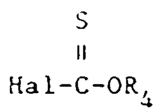 (IV)

où $R_4$ a la signification donnée pour la formule I et Hal représente un halogène ou en ce que l'on utilise, comme réactif approprié pour introduire un groupe β-thioéther dans un composé de formule IIb, un disulfure de formule V

$R_3$-SS-$R_3$ (V)

où $R_3$ a la significatin donnée pour la formule I.

**13.** Procédé selon la revendication 12, caractérisé en ce que la réaction avec les composés de formule III a lieu en présence d'une quantité catalytique d'un acide ou en présence à la fois d'une quantité catalytique d'un acide et d'un orthoester de formule VI $R_6C(OR_3)_3$ (VI) où $R_3$ a la signification donnée pour la formule I et $R_6$ représente l'hydrogène ou un alkyle en $C_1$-$C_6$, à des températures comprises entre -50° et + 150°C, de préférence, entre -20° et + 100°C.

**14.** Procédé selon la revendication 12, caractérisé en ce que l'on effectue la réaction avec les composés de formule IV dans un solvant inerte ou dans le réactif de formule IV à des températures comprises entre -50° et + 150°C, de préférence, allant de -20° à + 100°C, en présence d'une base.

**15.** Procédé selon la revendication 12 pour la préparation des composés de formule I où R représente le groupe β-mercapto, caractérisé en ce que l'on réduit un composé de formule I où R représente le groupe -S-C(O)$OR_4$, $R_4$ ayant le signification donnée pour la formule I, à des températures comprises entre 0° et 50°C pour former un composé 13β-mercapto de formule I.

**16.** Procédé selon la revendication 12, caractérisé en ce que l'on effectue la réaction avec un disulfure de formule V en présence d'une quantité au moins équimolaire d'une phosphine comportant trois liaisons et d'une quantité allant de 1/10 à 3 molaires d'un N-[$SR_3$]-sulfénimide où $R_3$ a la signification donnée pour la formule I, à des températures allant de 0° à +50°C.

**17.** Procédé pour la préparation des dérivés 13β-éther de formule I selon la revendication 1

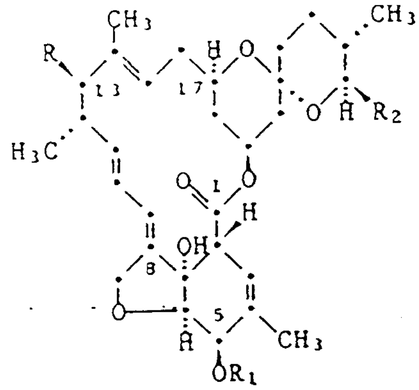

où R représente un reste $R_3$ lié par l'oxygène et $R_1$, $R_2$ et $R_3$ ont la signification selon la revendication 1, caractérisé en ce que l'on fait réagir un 13β -alcool de formule II

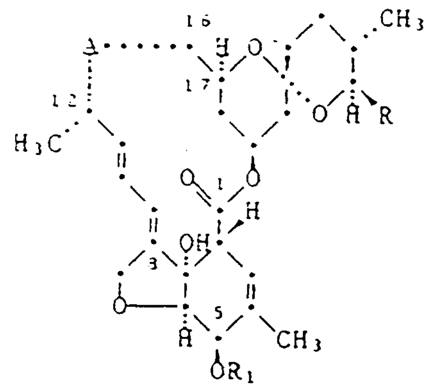

où A représente le groupe a

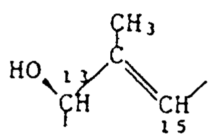 (a)

[= 13β-Hydroxy-$\Delta^{14,15}$]

$R_1$ représente un groupe protecteur et $R_2$ a la signification donnée pour la formule I, par une éthérification usuelle, de préférence, avec un alcool de formule III

$R_3$-XH (III)

où $R_3$ a la signification ci-dessus et X représente l'oxygène.

**18.** Procédé pour la préparation des dérivés 13$\beta$ -thioéther de formule I selon la revendication 1

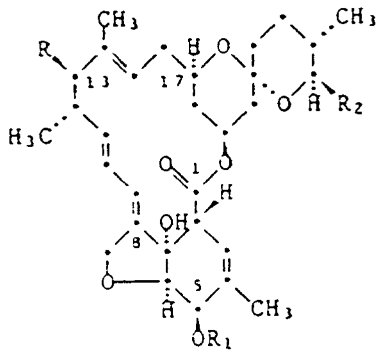

où R représente un reste $R_3$ lié par le soufre ou R représente le groupe -S-C(O)$OR_4$ et $R_1$, $R_2$, $R_3$ et $R_4$ ont la signification selon la revendication 1, caractérisé en ce que l'on thioéthérifie, de façon usuelle, un dérivé 13 $\beta$-mercapto de formule I où R représente le groupe 13 $\beta$-mercapto et les autres substituants sont définis comme ci-dessus, de préférence, par réaction avec un thiol de formule III

$R_3$-XH (III)

où $R_3$ a la signification ci-dessus et X représente le soufre.

**19.** Agent pour lutter contre les ectoparasites, endoparasites et insectes, caractérisé en ce qu'il contient, à côté des supports et/ou des agents de dispersion usuels, au moins un composé de formule I selon la revendication 1.

**20.** Agent selon la revendication 19, caractérisé en ce qu'il contient, comme composé de formule I, au moins un composé selon les revendications 2 à 10.

**21.** Procédé pour lutter contre les parasites sur les plantes, caractérisé en ce que l'on applique un composé de formule I selon l'une des revendications 1 à 10 sur la plante ou sur l'endroit où elle se trouve.

**22.** Procédé selon la revendication 21, caractérisé ce que les parasites sont des insectes.

**23.** Composé de formule I selon l'une des revendications 1 à 10 destiné à être utilisé dans un procédé pour lutter contre les ecto- et endoparasites chez l'animal.

**24.** Utilisation d'un composé de formule I selon l'une des revendications 1 à 10 pour la fabrication d'un produit pharmaceutique, de préférence, d'un produit pharmaceutique destiné à la médecine vétérinaire.